# EUROPEAN PATENT APPLICATION

(11) **EP 4 233 842 A2**
(43) Date of publication of application: **30.08.2023**
(21) Application number: 23151717.8
(22) Date of filing: 15.10.2019
(51) Int. Cl.: A61K 9/08, A61K 38/16, A61K 38/17, A61K 38/26, A61P 3/10, A61P 3/08, C07K 14/645, C07K 14/605

(54) **AVEXITIDE FOR THE TREATMENT OF HYPERINSULINEMIC HYPOGLYCEMIA**

(30) Priority: 15.10.2018 US 201862745915 P; 16.10.2018 US 201862746229 P; 25.03.2019 US 201962823493 P
(62) Divisional of application: 19873113.5
(71) Applicant: Eiger Biopharmaceuticals, Inc., Palo Alto, CA 94306 (US)
(72) Inventor: CRAIG, Colleen M., Palo Alto, California 94306 (US); PORTER, Lisa, Palo Alto, California 94306 (US)
(74) Representative: Patent Boutique LLP

(57) **Abstract**

Methods of treating hyperinsulinemic hypoglycemia (HH), including post-bariatric hypoglycemia (PBH) are provided. In some embodiments, the method comprises administering to a subject having HH a buffered liquid formulation composition.

## Description

### CROSS-REFERENCE

This application claims the benefit of priority to U.S. Provisional Application No. 62/745,915 (filed October 15, 2018), U.S. Provisional Application No. 62/746,229 (filed October 16, 2018), and U.S. Provisional Application No. 62/823,493 (filed March 25, 2019), each of which is incorporated herein by reference.

### REFERENCE TO A SEQUENCE LISTING SUBMITTED AS A TEXT FILE VIA EFS-WEB

The official copy of the sequence listing is submitted electronically via EFS-Web as an ASCII formatted sequence listing with a file named 1152075-002410PC_SL.TXT, created on October 11, 2019, and having a size of 735 bytes and is filed concurrently with the specification. The sequence listing contained in this ASCII formatted document is part of the specification and is herein incorporated by reference in its entirety.

### BACKGROUND

Insulin is a hormone secreted to control high blood glucose levels. Abnormal increases in insulin secretion can lead to profound hypoglycemia that can result in seizures, brain damage and death. Glucagon-like peptide-1 (GLP-1) is a gastrointestinal hormone that is released postprandially from intestinal L-cells and binds to GLP-1 receptors on beta cells of the pancreas, thereby enhancing insulin release.

Post-bariatric hypoglycemia (PBH), a form of hyperinsulinemic hypoglycemia (HH) is a rare disease with no approved pharmacotherapy, which is believed to be mediated primarily by exaggerated postprandial secretion and/or action of GLP-1 due to altered nutrient transit after bariatric surgery. PBH manifests as frequent, symptomatic, postprandial hypoglycemia that can lead to life-threatening neuroglycopenic consequences, such as loss of consciousness, seizures, cognitive dysfunction, and death. Quality of life can be severely diminished, and many patients cannot care for themselves or others, work, drive, or be left alone. To date, there are no approved pharmacotherapies for PBH.

Approximately 200,000-250,000 bariatric surgical procedures are performed in the United States each year. As the number of bariatric surgeries to treat severe obesity has increased, so too has the number of individuals who experience PBH. Accordingly, there is a growing unmet need for a therapy that safely and effectively mitigates hyperinsulinemic hypoglycemia and PBH.

### SUMMARY

In one aspect, methods of treating hyperinsulinemic hypoglycemia (HH), including post-bariatric hypoglycemia (PBH) are provided. In some embodiments, the method comprises subcutaneously administering to a subject having PBH a buffered liquid formulation comprising avexitide at a concentration of at least 30 mg/mL.

In some embodiments, the avexitide is administered at a total daily dose from about 40 mg to about 90 mg or from 30 mg to about 120 mg/day. In some embodiments, the avexitide is administered at a total daily dose of about 60 mg or 120 mg. In some embodiments, the avexitide is administered at a dose of about 45 mg BID or about 35 - 55 mg BID. In some embodiments, the avexitide is administered at a total daily dose of about 90 mg QD, or about 60 to 120 mg/day. In some embodiments, the avexitide is administered once daily (QD) or twice daily (BID).

In some embodiments, the avexitide is administered at a dose of 45 mg BID. In some embodiments, the BID dosing regimen comprises administering a morning dose and an evening dose and the evening dose is administered at least about 12 hours after the morning dose. In some embodiments, the morning dose is administered at least about 60 minutes before a meal. In certain embodiments, the avexitide is administered at 45 mg BID. In certain embodiments, a subject is administered avexitide from between about 30 mg to about 90 mg BID and wherein the subject is not required to fast after dosing or is not required to delay the first meal of the day, which includes having a first meal in the evening.

A benefit of BID dosing is that the subject is not no required to fast after dosing (or delay the first meal of the day), as trough concentrations are typically within the range of the concentration threshold (e.g., EC50) above which the therapeutic response is optimal.

In certain embodiments, the avexitide is administered at 45 mg - 120 QD. In certain embodiments, a subject is administered avexitide from between about 50 mg to about 100 mg QD and wherein the subject is required to fast after dosing or to delay the first meal of the day. In one embodiment, a subject is required to fast overnight before the morning dose. In one embodiment, subject is required to fast for approximately 45 - 90 minutes after the morning dose. In one embodiment, a subject is required to fast overnight before the morning dose and for approximately 45 - 90 minutes after the morning dose. The delay in the meal could be from about 30 minutes to about 1.5 hours. In some embodiments the delay is for 60 minutes, or from 45 - 60 minutes.

In some embodiments, the avexitide is administered at a dose of 90 - 120 mg QD. In some embodiments, the QD dosing regimen comprises administering the avexitide in the morning. In some embodiments, the avexitide is administered about 60 minutes before a meal, e.g., the first meal of the day. In some embodiments, the avexitide is administered between about 30 minutes to about 90 minutes before a meal. In certain embodiments, the avexitide is administered at 45 mg QD.

In some embodiments, the formulation comprises avexitide at a concentration from 30 mg/mL to 180 mg/mL. In some embodiments, the formulation comprises avexitide at a concentration of 30 mg/mL. In some embodiments, the formulation comprises avexitide at a concentration of 90 mg/mL. In some embodiments, the formulation comprises avexitide at a concentration of 100 - 120 mg/mL. In some embodiments, the formulation has a pH of about 5.5. In some embodiments, the formulation comprises a sodium acetate buffer. In some embodiments, the formulation comprises a tonicity modifier. In some embodiments, the tonicity modifier comprises mannitol.

In some embodiments, the subject has previously had a Roux-en-Y gastric bypass surgery. In some embodiments, the subject has previously had vertical sleeve gastrectomy (VSG) or biliopancreatic diversion (BPD) or other upper-GI procedure, such as gastrectomy +/- RYGB revision, esophagectomy, Nissen fundoplication.

In some embodiments, avexitide is administered for at least 30 days. In some embodiments, avexitide is administered for acute use for less than 30 days. In some embodiments, the avexitide is administered for less than 30 days and until one or more of the glucose and insulin levels are stabilized. In some embodiments, avexitide is administered for at least 45 days, or at least 90 days, or at least 180 days. In some embodiments, avexitide is administered for from between 30 days to 5 years.

In some embodiments, treatment reduces the number and/or severity of postprandial neuroglycopenic symptoms in the subject. In some embodiments, treatment improves postprandial glucose nadir. In some embodiments, treatment reduces postprandial insulin peak. In some embodiments, treatment reduces the rate of hypoglycemia in the subject. In some embodiments, treatment reduces the rate of severe hypoglycemia in the subject. In some embodiments, treatment reduces "severe hypoglycemia" (as defined by ADA - requiring 3rd party assistance). In other embodiments the treatment reduces the "rate of hypoglycemia-induced CNS impairment". In some embodiments, the administration of avexitide provides clinically meaningful improvement in one or more of the rate of hypoglycemia-induced CNS impairment, severe hypoglycemia, postprandial insulin peak, rate of severe hypoglycemia, or postprandial glucose nadir.

Further aspects and embodiments are disclosed *infra.*

### BRIEF DESCRIPTION OF THE DRAWINGS

The disclosure is described in detail below with reference to the appended drawings.
FIG. 1. Diagram of avexitide clinical study design according to aspects of this disclosure.
FIG. 2. Mean glucose concentrations (± standard deviation) during 180-minute MMTT for placebo, avexitide 30 mg BID, and avexitide 60 mg QD treatment groups according to aspects of this disclosure. The shaded bar across the bottom of the graph reflects glycemic rescue at glucose ≤50 mg/dL + neuroglycopenia or glucose ≤40 mg/dL +/- neuroglycopenia.
FIG. 3. Mean insulin concentrations (± standard deviation) during 180-minute MMTT for placebo, avexitide 30 mg BID, and avexitide 60 mg QD treatment groups according to aspects of this disclosure.
FIG. 4. Adverse events observed for placebo, avexitide 30 mg BID, and avexitide 60 mg QD treatment groups and overall adverse events observed according to aspects of this disclosure.
FIG. 5. Demonstrates that there is a significant reduction in hyperinsulinemic hypoglycemia and a reduced requirement for rescue in the 30 mg BID and 60 mg QD doses according to aspects of this disclosure.
FIG. 6. Demonstrates glycemic improvements in the outpatient setting showing that there is a reduction in diurnal percent time and number of episodes <70 and <55 mg/dL as measured by CGM according to aspects of this disclosure. In both graphs, for each of episodes <70 and <55 mg/dL, the left data bar is placebo, the middle data bar is 30 mg BID, and the right data bar is 60 mg QD.

### DETAILED DESCRIPTION

### Introduction

Avexitide is a 31-amino acid peptide that selectively targets and blocks GLP-1 receptors, normalizing insulin secretion by the pancreas, and thereby reducing postprandial hypoglycemia. Avexitide treatment reduces not only postprandial (protein-induced) but also fasting hyperinsulinemic hypoglycemia. Avexitide may also be used to treat insulinoma and hypoglycemia/NIPHS. As disclosed herein, in a clinical study investigating the safety and durability of effect of 28-day dosing of subcutaneous avexitide in subjects with refractory severe post-bariatric hypoglycemia, it was found that once daily and twice daily dosing regimens of avexitide improved postprandial glucose nadir, with fewer patients requiring glycemic rescue as compared to placebo dosing. Additionally, once daily and twice daily dosing regimens resulted in metabolic and clinical improvements in the patients, such as a reduced number of episodes of hypoglycemia.

### Definitions

The terminology used herein is for the purpose of describing particular embodiments only, and is not intended to be limiting. Unless defined otherwise, all technical and scientific terms used herein have the same meaning as commonly understood by one of ordinary skill in the art to which this invention belongs. In this specification and in the claims that follow, reference will be made to a number of terms that shall be defined to have the following meanings unless a contrary intention is apparent. In some cases, terms with commonly understood meanings are defined herein for clarity and/or for ready reference, and the inclusion of such definitions herein should not be construed as representing a substantial difference over the definition of the term as generally understood in the art.

Although any methods and materials similar or equivalent to those described herein can be used in the practice or testing of the present invention, the preferred methods and materials are now described. All technical and patent publications cited herein are incorporated herein by reference in their entirety.

All numerical designations, e.g., pH, temperature, time, concentration, and molecular weight, including ranges, are approximations which are varied ( + ) or ( - ) by increments of 0.1 or 1.0, as appropriate (e.g., pH 5.4 or 5.5). It is to be understood, although not always explicitly stated, that all numerical designations are preceded by the term "about." References to ranges include the end-points unless indicated otherwise. For example, administration of avexitide at a concentration of 30 mg/mL to 180 mg/mL includes administration of 30 mg/mL or 180 mg/mL.

The singular forms "a," "an," and "the" include plural referents unless the context clearly dictates otherwise. Thus, for example, reference to "a compound" includes a plurality of compounds.

The term "comprising" is intended to mean that the compounds, compositions and methods include the recited elements, but not excluding others. "Consisting essentially of" when used to define compounds, compositions and methods, means excluding other elements that would materially affect the basic and novel characteristics of the claimed invention. "Consisting of" means excluding any element, step, or ingredient not specified in the claim. Embodiments defined by each of these transition terms are within the scope of this invention.

"Avexitide," which is also referred to as "exendin (9-39)", refers to a 31 amino acid peptide with an empirical formula of C₁₄₉H₂₃₄N₄₀O₄₇S and a molecular weight of 3369.8 Daltons. Avexitide comprises residues 9-39 of the GLP-1 receptor agonist exendin-4 and is a GLP-1 receptor antagonist with inverse agonist properties. See Montrose-Rafizadeh et al., Journal of Biological Chemistry, 272:21201-21206 (1997). The amino acid sequence for avexitide is shown as follows: H-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂ (SEQ ID NO: 1). Avexitide has a predicted isoelectric point of 4.69 and has a net charge of -1 at pH 6 that increases to a net charge of +4 at pH 3.0. As used herein, the term "avexitide" also encompasses pharmaceutically acceptable salts of avexitide (exendin (9-39)), including but not limited to sulfate, hydrochloride, phosphate, sulfamate, acetate, citrate, lactate, tartrate, methanesulfonate, ethanesulfonate, benzenesulfonate, p-toluenesulfonate, cyclohexylsulfamate and quinate salts. In some embodiments, avexitide is in the form of an acetate or trifluoroacetate salt. Where not otherwise specified herein, avexitide acetate is used. Avexitide (exendin (9-39)) and pharmaceutically acceptable salts thereof are commercially available (e.g., Bachem).

The term "formulation" or "pharmaceutical formulation," as used herein, refers to a composition suitable for administration to a subject. A pharmaceutical formulation may be sterile, and preferably free of contaminants that are capable of eliciting an undesirable response within the subject (e.g., the compounds in the pharmaceutical formulation are pharmaceutical grade). Pharmaceutical formulations can be designed for administration to subjects or patients in need thereof via a number of different routes of administration, including oral, intravenous, buccal, rectal, parenteral, intraperitoneal, intradermal, intramuscular, subcutaneous, inhalational and the like. In some embodiments, a pharmaceutical formulation as described herein is formulated for subcutaneous administration.

As used herein, a "therapeutically effective amount" is an amount of an active ingredient (e.g., avexitide or its pharmaceutically acceptable salt) that eliminates, ameliorates, alleviates, or provides relief of the symptoms or leads to clinical outcomes for which it is administered.

The terms "treatment," "treating," and "treat," as used herein in reference to administering avexitide to treat HH, e.g., post-bariatric hypoglycemia, fasting hyperinsulinemic hypoglycemia, insulinoma and hypoglycemia/NIPHS, and covers any treatment of the disease in a human subject, and includes: (a) reducing the risk, frequency or severity of hypoglycemic episodes in patients with a history of post-bariatric hypoglycemia, (b) reducing the risk of occurrence of post-bariatric hypoglycemia in a subject determined to be predisposed to the disease, such as a person who has received post-bariatric surgery, but not yet diagnosed as having the disease, (c) impeding the development of the disease; and/or (d) relieving the disease, i.e., causing regression of the disease and/or relieving one or more disease symptoms.

The terms "administer," "administering," and "administration" as used herein, refer to introducing a compound (e.g., avexitide), a composition, or an agent into a subject or patient, such as a human. As used herein, the terms encompass both direct administration, (e.g., self-administration or administration to a patient by a medical professional) and indirect administration (e.g., the act of prescribing a compound or composition to a subject).

"QD" and "BID" have their usual meanings of administration of a composition (e.g., buffered liquid formulation of avexitide) once per day or twice per day, respectively. In some embodiments, administration once per day (QD) means that at least 20 hours, at least 22 hours, or about 24 hours elapse between administrations. In some embodiments, administration once per day means administration about every 24 hours. In some embodiments, administration twice per day (BID) means that at least 4 hours, at least 6 hours, at least 8 hours, at least 10 hours, at least 11 hours, or about 12 hours elapse between administrations. In some embodiments, administration twice per day means administration about every 12 hours.

As used herein, the terms "patient" and "subject" interchangeably refer to an individual (e.g., a human or a non-human mammal). In some embodiments, a patient or subject has post-bariatric hypoglycemia. In some embodiments, a patient or subject has previously had a bariatric procedure (e.g., gastric bypass surgery, vertical sleeve gastrectomy, biliopancreatic diversion). In some embodiments, a subject has previously had a different upper-gastrointestinal procedure (e.g., gastrectomy, esophagectomy, Nissen fundoplication). In some embodiments, a patient or subject has had vagotomy +/- pyloroplasty, bilroth, congenital microgastria. In other embodiments, a subject has a condition that speeds transit to the ileum (early nutrient exposure to L-cells). In some embodiments the subject has fasting hyperinsulinemic hypoglycemia. In some embodiments the subject has insulinoma. In some embodiments the subject has reactive hypoglycemia/NIPHS.

### Therapeutic Methods

In one aspect, methods of treating or preventing post-bariatric hypoglycemia (PBH) are provided. In some embodiments, the method comprising subcutaneously administering to a subject having PBH a composition comprising avexitide (e.g., a buffered liquid formulation as disclosed in Section IV below) at a concentration of at least 30 mg/mL. As described herein, in some embodiments, treatment with avexitide prevents or reduces one or more symptoms, metabolic outcomes, and/or clinical outcomes of post-bariatric hypoglycemia.

### Patient Population

In some embodiments, a subject to be treated according to the methods described herein is a subject having post-bariatric hypoglycemia (PBH) who has previously had a bariatric and/or related metabolic procedure. Bariatric and/or related metabolic procedures include, but are not limited to, Roux-en-Y Gastric Bypass, Vertical Sleeve Gastrectomy, placement of an endosleeve device, such as the EndoBarrier Gastrointestinal Liner System, also called an "endoluminal liner," duodenal mucosal resurfacing (also referred to as duodenal ablation), biliopancreatic diversion with duodenal switch, partial bypass of the duodenum, involving duodeno-ileal or duodeno-jejunal anastomosis, vagal nerve blockade, and pyloroplasty.

A bariatric procedure (e.g., bariatric surgery) typically involves any of the foregoing: partially or completely bypassing the duodenum and/or decreasing nutrient exposure to the duodenum, increasing the rapidity of nutrient transit to the lower part of the intestines (often specifically the ileum), and/or otherwise increasing ileal nutrient exposure. Bariatric surgery may be intended for weight loss or metabolic benefit (such as resolution of diabetes), or both. Such weight loss or metabolic procedures, referred to herein as "bariatric procedures" may enhance secretion of GLP-1 from the distal small intestine, especially the ileum, leading to elevated insulin secretion, and in some subjects, hypoglycemia. In some embodiments, the subject may be referred to as a "post bariatric surgery" subject.

In another embodiment, the subject to be treated has previously had a related procedure. As a non-limiting example, in one embodiment, the subject to be treated has previously had a non-bariatric surgical procedure involving the gastrointestinal system (including but not limited to esophagectomy, for example for treatment of esophageal cancer, Nissen Fundoplication, for example for treatment of gastroesophageal reflux, or gastrectomy, for example for treatment or prevention or treatment of gastric cancer) and so may be referred to herein as "post gastrointestinal surgery."

In some embodiments, the subject having hyperinsulinemic hypoglycemia has previously had a Roux-en-Y Gastric Bypass (RYGB) or Vertical Sleeve Gastrectomy (VSG). In some embodiments, the subject to be treated previously had a bariatric or related metabolic procedure (e.g., RYGB or VSG) at least one year prior to the onset of treatment.

Subjects with PBH may be identified by any suitable method. In some embodiments, PBH is diagnosed in a post-bariatric patient as the presence of symptomatic postprandial hypoglycemia associated with inappropriately elevated insulin (≥ 3 µU/mL) or C-peptide (> 0.6 ng/mL) at the time of hypoglycemia (< 54 mg/dL glucose). In some embodiments, PBH is diagnosed by the presence of Whipple's triad in a post-bariatric patient, which has the following criteria: (1) the occurrence of hypoglycemic symptoms (e.g., neuroglycopenic symptoms such as behavioral changes, confusion or impaired cognitive function, seizure, loss of consciousness); (2) documented low blood glucose level at the time of the symptoms (e.g., < 54 mg/dL; <70 mg/dL); and (3) resolution of the symptoms after blood glucose is raised (e.g., within a few minutes). In some embodiments, PBH is diagnosed using a positive provocative test, e.g., an oral glucose tolerance test (OGTT) or a mixed meal tolerance test (MMTT). Diagnostic criteria for PBH is also disclosed in Salehi et al., The Journal of Clinical Endocrinology & Metabolism, 2018, 103:2815-2826.

In some embodiments, the subject has refractory PBH. As used herein, a subject or patient having "refractory PBH" refers to a PBH patient who is undergoing conventional treatment, e.g., dietary modification, or the use of rescue glucagon who still experiences (1) hypoglycemia that interferes with the patient's daily activities, and/or (2) severe hypoglycemic episodes.

In some embodiments, the subject has severe PBH. As used herein, a subject or patient having "severe PBH" refers to a PBH patient who experiences severe hypoglycemia. "Severe hypoglycemia," as used herein, is defined as hypoglycemic symptoms confirmed by self blood glucose monitoring (SBGM) glucose ≤ 54 mg/dL (see IHSG 2017). As used herein severe hypoglycemia can also be a severe event characterized by altered mental and/or physical functioning that requires assistance from another person for recovery. (ADA, Diabetes Care 2019;42(Suppl. 1):561-S70). In one embodiment, the rate of severe hypoglycemia episodes (e.g., hypoglycemia-induced CNS impairment) is reduced. If SBGM is not obtained at the time of symptoms or if the patient does not experience symptoms due to hypoglycemia unawareness, severe hypoglycemia may be documented based on a corresponding continuous glucose monitor (CGM) measure ≤ 54 and/or neurological recovery following the return of glucose to normal. "Severe hypoglycemia" can also refer to a hypoglycemic episode that requires assistance of another person to administer rescue therapy (see Seaquist 2013).

Suitable patient populations and methods of identifying patients are also described in PCT Patent Application Nos. PCT/US2016/033837 and PCT/US2017/062838, incorporated in their entirety by reference herein.

In some embodiments, the patient is a human patient. In some embodiments, the patient is an adult. In some embodiments, the patient is a juvenile.

### Dosing Regimens

In some embodiments, avexitide is administered at a total daily dose in the range of about 40 mg to about 90 mg. For PBH and other upper-GI types of HH, dosing in adults will be about 45 mg BID, or from 30 mg BID-60 mg BID, or from 60 mg QD-120 mg QD., e.g., a total daily dose of avexitide from about 40 mg to about 75 mg, from about 40 mg to about 60 mg, from about 50 mg to about 90 mg, from about 45 mg to about 75 mg, from about 60 mg to about 90 mg, or from about 60 mg to about 75 mg. In some embodiments, avexitide is administered at a total daily dose of at least about 40 mg, e.g., at least about 45 mg, at least about 50 mg, at least about 60 mg, or at least about 75 mg. In some embodiments, avexitide is administered at a total daily dose of about 40 mg, about 45 mg, about 50 mg, about 55 mg, about 60 mg, about 65 mg, about 70 mg, about 75 mg, about 80 mg, about 85 mg, or about 90 mg.

In some embodiments, the subjects will receive 0.375 mg/kg/dose or 0.75 mg/kg/day. In some embodiments, the subjects will receive about 0.56 mg/kg/dose or about 1.125 mg/kg/day.

In some embodiments, the subjects will receive 0.3 - 1.6 mg/kg/dose of avexitide BID.

In some embodiments, the subjects will receive about 0.6-3.2 mg/kg/day BID or about 0.2 -1.2 mg/kg/dose, or about 0.6-3.6 mg/kg/day BID or TID. In some embodiments, subjects are administered 0.6-3.6 mg/kg/day TID.

In some embodiments, the avexitide will be administered by IV infusion and will be administered a from about 0.1 to 1.0 mg/kg/hr, or from about 0.2 to 0.6 mg/kg/hr.

The infusion formulation of avexitide may also include albumin to prevent peptide sticking to tubing/bag, and may be diluted in for example normal saline or other diluent; may include low absorbing tubing and bag.

The dosage ranges described above are exemplary adult doses, neonate and child doses, and may vary depending upon the age and weight of the patient as would be known by those skilled in the pharmaceutical arts. It will be appreciated that in some embodiments, dosage may be increased or decreased during the course of treatment. For example, some physicians may desire to treat with a low or initiating (starting) dose, escalate to an increased dose if the initiating dose does not provide sufficient therapeutic benefit, and maintain the initiating dose if the initiating dose provides sufficient therapeutic benefit.

In some embodiments, a therapeutically effective amount of avexitide (or a pharmaceutically acceptable salt thereof) is administered once daily (QD). QD administration is well-known in the medical arts. In some embodiments QD doses are administered (e.g., self-administered) at about 24 hour intervals (e.g., 7 a.m. on successive days). However, shorter (e.g., 8 a.m. and 6 a.m. on successive days) or longer (e.g., 7 a.m. and 9 a.m. on successive days) intervals between administration are possible provided the administrations are at least about 18 hours apart. Preferably, the administrations are at least about 20 hours, 21 hours, 22 hours, 23 hours, or 24 hours apart. Preferably, the administrations are not more than 30 hours apart. Although administration once a day is preferred, the dosage administered can occur more frequently (e.g., two times a day) or less frequently (e.g., once every other day).

In some embodiments, the avexitide formulation is administered twice daily (BID). BID (twice per day) administration is well known in the medical arts. The formulation can be administered at specific points in the day or schedule of a subject, e.g., morning, afternoon, evening, night, before or during or after meals, before bedtime, etc. In some embodiments, the formulation is administered about once every 12 hours. In some embodiments BID doses are administered (e.g., self-administered) at about 12 hour intervals (e.g., 7 a.m. and 7 p.m.). However, shorter (e.g., 8 a.m. and 6 p.m.) or longer (e.g., 7 a.m. and 10 p.m.) intervals between administrations are possible. In some embodiments, the administrations are at least about 4 hours, 6 hours, 7 hours, 8 hours, 9 hours, 10 hours or 11 hours apart. Preferably the administrations are not more than about 15 hours apart. Methods of timing the administration of BID dosing are described, for example, in PCT Patent Application No. PCT/US2016/033837, incorporated by reference herein.

In some embodiments, the avexitide formulation is administered (e.g., subcutaneously or i.v. or s.c. infusion, administered) at a dose of from about 20 - 65 mg BID or from 20-45 mg BID. In some embodiments, the avexitide formulation is administered at a dose of about 20 mg BID. In some embodiments, the avexitide formulation is administered at a dose of about 25 mg BID. In some embodiments, the avexitide formulation is administered at a dose of about 30 mg BID. In some embodiments, the avexitide formulation is administered at a dose of about 35 mg BID. In some embodiments, the avexitide formulation is administered at a dose of about 40 mg BID. In some embodiments, the avexitide formulation is administered at a dose of about 45 mg BID. In some embodiments, the avexitide formulation is administered at a dose of about 50 mg BID. In some embodiments, the avexitide formulation is administered at a dose of about 55 mg BID. In some embodiments, the avexitide formulation is administered at a dose of about 60 mg BID. In some embodiments, the avexitide formulation is administered at a dose of about 65 mg BID. In some embodiments, a formulation comprising avexitide at a concentration of 30 mg/ml or higher is administered at a dose of about 20 - 65 mg BID, e.g., at a dose of about 30 mg BID. In some embodiments, a formulation comprising avexitide at a concentration of between about 15 mg/ml and 100 mg/ml or between about 2 mg/ml to about 100 mg/ml. In some embodiments, a formulation comprising avexitide at a concentration of 90 mg/ml.

In some embodiments, the avexitide formulation is administered (e.g., subcutaneously administered) at a total daily dose of 40-60 mg or from 100 or 120 mg and is administered BID. In some embodiments, the avexitide formulation is administered at a total daily dose of 40 mg and is administered BID (i.e., 20 mg BID). In some embodiments, the avexitide formulation is administered at a total daily dose of 50 mg and is administered BID (i.e., 25 mg BID). In some embodiments, the avexitide formulation is administered at a total daily dose of 60 mg and is administered BID (i.e., 30 mg BID); the avexitide formulation is administered at a total daily dose of 120 mg and is administered BID (i.e., 60 mg BID)

In some embodiments, the avexitide formulation is administered (e.g., subcutaneously administered) at a dose of about 40-90 mg QD, e.g., about 60-90 mg QD or about 45-75 mg QD. In some embodiments, the avexitide formulation is administered at a dose of about 40 mg QD. In some embodiments, the avexitide formulation is administered at a dose of about 50 mg QD. In some embodiments, the avexitide formulation is administered at a dose of about 60 mg QD. In some embodiments, the avexitide formulation is administered at a dose of about 75 mg QD. In some embodiments, the avexitide formulation is administered at a dose of about 90 mg QD. In some embodiments, the avexitide formulation is administered at a dose of about 100 mg QD. In some embodiments, the avexitide formulation is administered at a dose of about 110 mg QD. In some embodiments, the avexitide formulation is administered at a dose of about 120 mg QD. In some embodiments, the avexitide formulation is administered at a dose of about 125 mg QD. In some embodiments, a formulation comprising avexitide at a concentration of 30 mg/ml or higher is administered at a dose of about at 45-90 mg QD, e.g., a dose of about 60 mg QD, about 75 mg QD, about 80 QD, about 85 QD, about 90 QD, about 100 QD, about 110 QD or about 120 QD or from about 45 QD to about 120 QD.

In some embodiments, the avexitide formulation is administered (e.g., subcutaneously administered) at a total daily dose of 60-90 mg and is administered QD. In some embodiments, the avexitide formulation is administered at a total daily dose of 60 mg and is administered QD (i.e., 60 mg QD). In some embodiments, the avexitide formulation is administered at a total daily dose of 70 mg and is administered QD (i.e., 70 mg QD). In some embodiments, the avexitide formulation is administered at a total daily dose of 75 mg and is administered QD (i.e., 75 mg QD). In some embodiments, the avexitide formulation is administered at a total daily dose of 80 mg and is administered QD (i.e., 80 mg QD). In some embodiments, the avexitide formulation is administered at a total daily dose of 90 mg and is administered QD (i.e., 90 mg QD). In some embodiments, the avexitide formulation is administered at a total daily dose of 120 mg and is administered QD (i.e., 120 mg QD).

In some embodiments, the avexitide formulation is administered (e.g., subcutaneously administered) at a total daily dose of 60-90 mg and is administered BID or QD. In some embodiments, the avexitide formulation is administered at a total daily dose of 60 mg and is administered QD (i.e., 60 mg QD). In some embodiments, the avexitide formulation is administered at a total daily dose of 60 mg and is administered BID (i.e., 30 mg BID). In some embodiments, the avexitide formulation is administered at a total daily dose of 70 mg and is administered QD (i.e., 70 mg QD). In some embodiments, the avexitide formulation is administered at a total daily dose of 70 mg and is administered BID (i.e., 35 mg BID). In some embodiments, the avexitide formulation is administered at a total daily dose of 80 mg and is administered QD (i.e., 80 mg QD). In some embodiments, the avexitide formulation is administered at a total daily dose of 80 mg and is administered BID (i.e., 40 mg BID). In some embodiments, the avexitide formulation is administered at a total daily dose of 90 mg and is administered QD (i.e., 90 mg QD). In some embodiments, the avexitide formulation is administered at a total daily dose of 90 mg and is administered BID (i.e., 45 mg BID). In some embodiments, the avexitide formulation is administered at a total daily dose of 120 mg and is administered QD (i.e., 120 mg QD). In some embodiments, the avexitide formulation is administered at a total daily dose of 90 mg and is administered BID (i.e., 60 mg BID).

In some embodiments, the avexitide formulation is administered (e.g., subcutaneously administered) twice daily (BID) within about 60 minutes prior to morning and evening meals (or prior to the two main meals of the day). In some embodiments, the avexitide formulation is administered at least about 60 minutes prior to a meal (e.g., at least 60 minutes prior to a morning meal and/or at least 60 minutes prior to an evening meal). In some embodiments, the administrations prior to the morning and evening meals (or prior to the two main meals of the day) are at least about 6 hours apart. In some embodiments, the administration of the formulation is not timed to meals.

In some embodiments, the avexitide formulation is administered (e.g., subcutaneously administered) once daily (QD) in the morning or at night to maximally cover the morning and evening meals. For example, in some embodiments, the formulation is administered at night after the evening meal or early in the morning prior to the morning meal (e.g., at least 60 minutes prior to the morning meal).

### Routes of Administration

In some embodiments, a composition comprising avexitide (e.g., a buffered liquid formulation as disclosed in Section IV below) is administered to a subject by subcutaneous administration (e.g., subcutaneous injection). Sites of injection or administration or s.c. infusion, include, but are not limited to, injection in the thigh, abdomen, upper arm region, or upper buttock region.

In some embodiments, a composition comprising avexitide (e.g., a buffered liquid formulation as disclosed in Section IV below) is formulated for subcutaneous administration. In one embodiment, the avexitide composition is formulated for subcutaneous administration according to a once daily (QD) or twice daily (BID) regime.

### Avexitide Formulations

In some embodiments, the therapeutic methods disclosed herein comprise administering a buffered liquid formulation comprising avexitide or a pharmaceutically acceptable salt thereof. In some embodiments, the formulation comprises avexitide at a concentration of at least 30 mg/mL.

In some embodiments, the formulation comprises avexitide at a concentration of about 2-180 mg/mL, about 2-90 mg/mL, about 2-20 mg/mL, about 2-29 mg/ml, about 1-20 mg/ml, about 30-150 mg/mL, about 30-120 mg/mL, about 30-90 mg/mL, about 30-60 mg/mL, about 30-70 mg/mL, about 40-180 mg/mL, about 40-120 mg/mL, about 45-90 mg/mL, about 45-75 mg/mL, about 60-180 mg/mL, about 60-120 mg/mL, about 60-90 mg/mL, or about 30-70 mg/mL (e.g., about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 110 mg/mL, about 120 mg/mL, about 130 mg/mL, about 140 mg/mL, about 150 mg/mL, about 160 mg/mL, about 170 mg/mL, or about 180 mg/mL). In some embodiments, the formulation comprises avexitide at a concentration of about 30 mg/ml. In some embodiments, the formulation comprises avexitide at a concentration in the range of about 40 mg/mL to about 50 mg/mL. In some embodiments, the formulation comprises avexitide at a concentration of about 45 mg/mL. In some embodiments, the formulation comprises avexitide at a concentration in the range of about 30 mg/mL to about 60 mg/mL. In some embodiments, the formulation comprises avexitide at a concentration in the range of about 30 mg/mL to about 90 mg/mL. In some embodiments, the formulation comprises avexitide at a concentration in the range of about 45 mg/mL to about 90 mg/mL. In some embodiments, the formulation comprises avexitide at a concentration of about 60 mg/mL. In some embodiments, the formulation comprises avexitide at a concentration of about 75 mg/mL. In some embodiments, the formulation comprises avexitide at a concentration of about 90 mg/mL.

### Duration of Treatment and Treatment Endpoints

Subjects may receive avexitide therapy for a predetermined time, an indefinite time, or until an endpoint is reached. Treatment may be continued on a continuous daily or weekly basis for at least two to three months, six months, one year, or longer. In some embodiments, therapy is for at least 30 days, at least 60 days, at least 90 days, at least 120 days, at least 150 days, or at least 180 days. In some embodiments, treatment is continued for at least 6 months, at least 7 months, at least 8 months, at least 9 months, at least 10 months, at least 11 months, or at least one year. In some embodiments, treatment is continued for the rest of the patient's life or until administration is no longer effective to provide meaningful therapeutic benefit.

In one embodiment, the subject will be treated for less than 30 days to stabilize the glucose and insulin levels.

In some embodiments, treatment with avexitide therapy as disclosed herein results in an improvement in postprandial hypoglycemia. For example, in some embodiment, treatment with avexitide therapy results in an increase in postprandial plasma glucose nadir in the subject as compared to baseline (e.g., as compared to the subject's postprandial plasma glucose nadir prior to the onset of treatment, e.g., as measured during MMTT). In some embodiments, treatment with avexitide therapy as disclosed herein results in an improvement in postprandial neuroglycopenic symptoms or hypoglycemic symptoms.

In some embodiments, treatment reduces the number and/or severity of postprandial neuroglycopenic symptoms in the subject. In some embodiments, treatment improves postprandial glucose nadir. In some embodiments, treatment reduces postprandial insulin peak. In some embodiments, treatment reduces the rate of hypoglycemia in the subject. In some embodiments, treatment reduces the rate of severe hypoglycemia in the subject. In some embodiments, treatment reduces "severe hypoglycemia" (as defined by ADA - requiring 3rd party assistance). In other embodiments the treatment reduces the "rate of hypoglycemia-induced CNS impairment". In some embodiments, the administration of avexitide provides clinically meaningful improvement in one or more of the rate of hypoglycemia induced CNS impairment, severe hypoglycemia, postprandial insulin peak, rate of severe hypoglycemia, or postprandial glucose nadir.

In some embodiments, the avexitide formulation is administered (e.g., subcutaneously administered) twice daily (BID) within about 60 minutes prior to morning and evening meals (or prior to the two main meals of the day). In some embodiments, the avexitide formulation is administered at least about 60 minutes prior to a meal (e.g., at least 60 minutes prior to a morning meal and/or at least 60 minutes prior to an evening meal). In some embodiments, the administrations prior to the morning and evening meals (or prior to the two main meals of the day) are at least about 6 hours apart. In some embodiments, the administration of the formulation is not timed to meals.

In some embodiments, the avexitide formulation is administered (e.g., subcutaneously administered) once daily (QD) in the morning or at night to maximally cover the morning and evening meals. For example, in some embodiments, the formulation is administered at night after the evening meal or early in the morning prior to the morning meal (e.g., at least 60 minutes prior to the morning meal).

For example, in some embodiments, treatment with avexitide therapy results in a reduction in the number and/or severity of postprandial neuroglycopenic symptoms as compared to baseline (e.g., as compared to the number and/or severity of postprandial neuroglycopenic symptoms in the subject prior to the onset of treatment, e.g., as measured during MMTT). In some embodiments, treatment with avexitide therapy as disclosed herein results in an improvement in postprandial insulin response. For example, in some embodiments, treatment with avexitide therapy results in a decrease in peak postprandial insulin concentration as compared to baseline (e.g., as compared to the subject's peak postprandial insulin concentration prior to the onset of treatment, e.g., as measured during MMTT). In some embodiments, treatment with avexitide therapy as disclosed herein results in an improvement in the patient's rate of hypoglycemia and/or rate of severe hypoglycemia. For example, in some embodiments, treatment with avexitide therapy results in a reduction in the rate of hypoglycemia and/or a reduction in the rate of severe hypoglycemia as compared to baseline or placebo or other control (e.g., as compared to the subject's rate of hypoglycemia and/or rate of severe hypoglycemia, e.g., as measured by self blood glucose monitoring or continuous glucose monitoring). Treatment endpoints and methods of measuring treatment endpoints are also described in the Examples section below, e.g., at Table 1.

### Physiologically Acceptable Buffers

In some embodiments, the formulation comprises avexitide or a pharmaceutically acceptable salt thereof in a physiologically acceptable buffer having a pH in the range of about 5 to about 6. In some embodiments, the buffer is compatible with subcutaneous administration. In some embodiments, the physiologically acceptable buffer is a buffer that results in a liquid formulation having a pH at or about physiological pH, or within a relatively narrow pH range near physiological pH (e.g., between about 5.0 to about 8.0). In one embodiment, the buffered liquid formulation comprises a physiologically acceptable buffer having a pH above 5.0 and up to about 6. In some embodiments, the physiologically acceptable buffer has a pH above 5.0 and up to about 5.5. In one embodiment, the physiologically acceptable buffer has a pH in the range of 5.2 to 5.8 (e.g., 5.2, 5.3, 5.4, 5.5, 5.6, 5.7, or 5.8). In one embodiment, the physiologically acceptable buffer has a pH in the range of 5.0 to 5.5 (e.g., 5.1, 5.2, 5.3, 5.4, or 5.5). In one embodiment, the physiologically acceptable buffer has a pH in the range of about 5.5 to about 6. In one embodiment, the physiologically acceptable buffer has a pH of about 5.5.

In one embodiment, the physiologically acceptable buffer comprises an acetate buffer, a citrate buffer, a phosphate buffer, a histidine buffer, or a mixture thereof. In one embodiment, the physiologically acceptable buffer comprises sodium acetate, potassium acetate, trisodium citrate, magnesium citrate, potassium citrate, potassium phosphate, or a mixture thereof. In one embodiment, the physiologically acceptable buffer comprises a buffering agent (e.g., sodium acetate) at a concentration from about 5 mM to about 30 mM, about 10 mM to about 30 mM, about 15 mM to about 30 mM, about 20 mM to about 30 mM, or about 25 mM to about 30 mM (e.g., about 5 mM, about 8 mM, about 10 mM, about 12 mM, about 15 mM, about 18 mM, about 20 mM, about 22 mM, about 25 mM, about 28 mM, or about 30 mM). In some embodiments, the physiologically acceptable buffer comprises the buffering agent (e.g., sodium acetate) at a concentration of at least 10 mM.

In some embodiments, the physiologically acceptable buffer comprises an acetate buffer. In some embodiments, the buffering agent is sodium acetate. In some embodiments, the buffering agent is potassium acetate. In some embodiments, the physiologically acceptable buffer comprises an acetate buffer (e.g., sodium acetate or potassium acetate) at a concentration of about 5 mM to about 30 mM, e.g., about 10 mM to about 20 mM. In some embodiments, the physiologically acceptable buffer comprises an acetate buffer (e.g., sodium acetate or potassium acetate) at a concentration of about 10 mM.

In some embodiments, the physiologically acceptable buffer comprises a citrate buffer. In some embodiments, the buffering agent is trisodium citrate. In some embodiments, the buffering agent is magnesium citrate. In some embodiments, the buffering agent is potassium citrate. In one embodiment, the physiologically acceptable buffer comprises the citrate buffer (e.g., sodium citrate, magnesium citrate, or potassium citrate) at a concentration from about 5 mM to about 30 mM, e.g., about 10 mM to about 20 mM. In some embodiments, the physiologically acceptable buffer comprises the citrate buffer (e.g., sodium citrate, magnesium citrate, or potassium citrate) at a concentration of about 10 mM.

In one embodiment, the physiologically acceptable buffer comprises a phosphate buffer. In one embodiment, the physiologically acceptable buffer comprises potassium phosphate. In one embodiment, the physiologically acceptable buffer comprises potassium phosphate at a concentration from about 5 mM to about 30 mM, e.g., about 10 mM to about 20 mM. In some embodiments, the physiologically acceptable buffer comprises the phosphate buffer (e.g., potassium phosphate) at a concentration of about 10 mM.

### Tonicity Modifiers

In some embodiments, the buffered formulation comprises a tonicity modifier. In some embodiments, the tonicity modifier is mannitol, dextrose, glycerin, lactose, sucrose, trehalose, or a mixture thereof. In some embodiments, the tonicity modifier is mannitol. The use of tonicity modifiers is well known in the medicinal arts, and one of skill in the art can use one or more of the tonicity modifiers disclosed herein to provide a liquid pharmaceutical formulation suitable for subcutaneous administration. See, for example, Pramanick et al., Pharma Times., Vol 45, No. 3, (2013); see also, Formulating Poorly Water Soluble Drugs, Williams, Watts, and Miller, eds., Springer Science and Business Media (2011).

In some embodiments, the tonicity modifier or combination of tonicity modifiers is present in the formulation at a concentration of about 20-75 mg/ml, about 20-60 mg/ml, about 25-55 mg/ml, about 30-75 mg/ml, about 30-50 mg/ml, about 35-45 mg/ml, about 40-45 mg/ml, about 45-75 mg/ml, or about 45-60 mg/ml (e.g., about 20 mg/ml, about 22 mg/ml, about 25 mg/ml, about 28 mg/ml, about 30 mg/ml, about 32 mg/ml, about 35 mg/ml, about 38 mg/ml, about 40 mg/ml, about 42 mg/ml, about 45 mg/ml, about 48 mg/ml, about 50 mg/ml, about 52 mg/ml, about 55 mg/ml, about 58 mg/ml, about 60 mg/ml, about 65 mg/ml, about 70 mg/ml, or about 75 mg/ml). In some embodiments, the formulation comprises the tonicity modifier at a concentration range of about 30 mg/ml to about 60 mg/ml.

In some embodiments, the tonicity modifier comprises mannitol. In some embodiments, the mannitol is present at a concentration of about 40-50 mg/ml. In some embodiments, the mannitol is present at a concentration in the range of about 40 mg/ml to about 45 mg/ml. In some embodiments, the mannitol is present at a concentration of about 45 mg/ml. In some embodiments, the mannitol is present at a concentration of at least 45 mg/ml.

In one embodiment, the tonicity modifier comprises dextrose. In one embodiment, the dextrose is present at a concentration of about 20 mg/ml to about 60 mg/ml (e.g., about 20 mg/ml, about 40 mg/ml, about 45 mg/ml, or about 60 mg/ml).

In one embodiment, the tonicity modifier comprises glycerin. In some embodiments, the glycerin is present at a concentration of about 20 mg/ml to about 60 mg/ml (e.g., about 20 mg/ml, about 40 mg/ml, about 45 mg/ml, or about 60 mg/ml).

In one embodiment, the tonicity modifier comprises lactose. In one aspect, the lactose is present at a concentration of about 20 mg/ml to about 60 mg/ml (e.g., about 20 mg/ml, about 40 mg/ml, about 45 mg/ml, or about 60 mg/ml).

In one embodiment, the tonicity modifier comprises sucrose. In some embodiments, the sucrose is present at a concentration of about 20 mg/ml to about 60 mg/ml (e.g., about 20 mg/ml, about 40 mg/ml, about 45 mg/ml, or about 60 mg/ml).

In one embodiment, the tonicity modifier comprises trehalose. In one aspect, the trehalose is present at a concentration of about 20 mg/ml to about 60 mg/ml (e.g., about 20 mg/ml, about 40 mg/ml, about 45 mg/ml, or about 60 mg/ml).

In some embodiments, the buffered formulation comprises two or more tonicity modifiers. In some embodiments, the buffered formulation comprises two or more tonicity modifiers selected from the group consisting of mannitol, dextrose, glycerin, lactose, sucrose, and trehalose. In some embodiments, the buffered formulation comprises mannitol at least one other tonicity modifier.

### Additional Excipients

In some embodiments, the formulation further comprises one or more additional excipients such as preservatives, surfactants (e.g., a polysorbate or a poloxamer), or colorants (e.g., pharmaceutically acceptable dyes, inorganic pigments, and natural colorants). A wide variety of pharmaceutically acceptable excipients are known in the art. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A.H. Kibbe et al., eds., 3rd ed. Amer. Pharmaceutical Assoc., each of which is incorporated by reference herein.

In some embodiments, the avexitide is formulated as disclosed in International Application Publication No. WO 2018/094404, incorporated by reference herein.

### Injectate Formulations

In some embodiments, the avexitide formulation is provided as a single-use prefilled syringe, e.g., in a kit comprising multiple single-use prefilled syringes (e.g., 10, 20, 30, 40, 50, or 60 prefilled syringes). In some embodiments, the single-use prefilled syringe comprises an avexitide formulation comprising avexitide at a concentration of at least 30 mg/mL, a tonicity modifier, and a buffer having a pH in the range of 5.0 to 6.0. In some embodiments, the single-use prefilled syringe comprises avexitide in an amount of at least 20 mg (e.g., 20-90 mg, 30-75 mg, 30-60 mg, 40-90 mg, or 60-90 mg, e.g., 20 mg, 25 mg, 30 mg, 40 mg, 45 mg, 50 mg, 60 mg, 70 mg, 80 mg, or 90 mg). In some embodiments, the single-use prefilled syringe comprises avexitide in an amount of at least 30 mg. In some embodiments, the avexitide formulation optionally further comprises a preservative.

In some embodiments, the avexitide formulation is provided in a pen injector device. In some embodiments, the pen injector device is a glass device (e.g., a glass cartridge pen injector device). In some embodiments, the pen injector device is a single-use device. In some embodiments, the pen injector device comprises an avexitide formulation comprising avexitide at a concentration of at least 30 mg/mL, a tonicity modifier, and a buffer having a pH in the range of 5.0 to 6.0. In some embodiments, the pen injector device comprises avexitide in an amount of at least 20 mg (e.g., 20-90 mg, 30-75 mg, 30-60 mg, 40-90 mg, or 60-90 mg, e.g., 20 mg, 25 mg, 30 mg, 40 mg, 45 mg, 50 mg, 60 mg, 70 mg, 80 mg, or 90 mg). In some embodiments, the pen injector device comprises avexitide in an amount of at least 30 mg. As a non-limiting example, the pen injector device comprises a formulation that comprises about 30-75 mg of avexitide (e.g., about 30 mg or about 60 mg of avexitide), a tonicity modifier and a buffer having a pH in the range of about 5.0 to 6.0. In some embodiments, the avexitide formulation optionally further comprises a preservative.

In some embodiments, the avexitide formulation is provided in a unit or multi-dose glass vial or ampule for administration with the use of a syringe, similar to a glucagon emergency kit. In some embodiments, the avexitide formulation is provided as an injectable solution in a single-dose tray containing a vial of an avexitide formulation as described herein (e.g., a formulation comprising about 20-90 mg of avexitide (e.g., 30-75 mg, 30-60 mg, 40-50 mg, 40-90 mg, or 60-90 mg of avexitide), a tonicity modifier, and a buffer having a pH in the range of 5.0 to 6.0, and optionally an appropriate volume of an antimicrobial preservative), a vial connector, a syringe, and one or more needles.

In some embodiments, each dose is administered in a total volume ranging from 0.25-2 ml inj ectate. In some embodiments, each dose is administered in a total volume ranging from about 0.1 ml or even 0.05 ml, with most subjects administering an injection volume ranging from 0.25-1.5 ml, or from 0.5-1 ml, or from 0.7-1 ml, or from 0.1 ml or even 0.05 ml.

As used below, any reference to a series of embodiments is to be understood as a reference to each of those embodiments disjunctively (e.g., "Embodiments 1-4" is to be understood as "Embodiments 1, 2, 3, or 4").

Embodiment 1 is a method of treating one or more of hyperinsulinemic hypoglycemia (HH), fasting hyperinsulinemic hypoglycemia, insulinoma, or hypoglycemia/NIPHS, the method comprising subcutaneously administering to the subject a buffered liquid formulation comprising avexitide at a concentration of between 2 - 90 mg/mL.

Embodiment 2 is the method of embodiment 1, wherein the avexitide is administered less than 30 days.

Embodiment 3 is the method of embodiment(s) 1 and 2, wherein the avexitide is administered until one or both of glucose level and insulin level are stabilized.

Embodiment 4 is the method of any of embodiment(s) 1-3, wherein HH comprises post-bariatric hypoglycemia (PBH).

Embodiment 5 is the method of any of embodiment(s) 1-4, wherein the subject has previously had one or more of Roux-en-Y gastric bypass surgery, vertical sleeve gastrectomy (VSG), biliopancreatic diversion (BPD), upper-GI procedure, gastrectomy, esophagectomy, Nissen fundoplication, gastric bypass surgery, vagotomy with pyloroplasty, vagotomy without pyloroplasty, or bilroth.

Embodiment 6 is the method of any of embodiment(s) 1-3, wherein the subject has congenital microgastria or a condition that speeds transit to the ileum.

Embodiment 7 is the method of any of embodiment(s) 1-6, wherein the avexitide is administered at a total daily dose from about 40 mg to about 90 mg.

Embodiment 8 is the method of any of embodiment(s) 1-6, wherein the avexitide is administered at a total daily dose from about 40 mg to about 120 mg; or from 30 mg - 60 mg BID, or from 60 mg - 120 mg QD; or from about 45 mg BID, or about 35 - 55 mg BID.

Embodiment 9 is the method of any of embodiment(s) 1-6, wherein the avexitide is administered at a total daily dose is about 90 mg; or about 90 mg QD, or about 60 to 120 mg/day.

Embodiment 10 is the method of any of embodiment(s) 1-6, wherein the subject is administered 0.375 mg/kg/dose or 0.56 mg/kg/dose or 0.75 mg/kg/day or about 1.125 mg/kg/day, or about 0.3 - 1.6 mg/kg/dose of avexitide BID; or about 0.6-3.2 mg/kg/day BID or about 0.2 -1.2 mg/kg/dose, or about 0.6-3.6 mg/kg/day BID; or about 0.6-3.6 mg/kg/day TID.

Embodiment 11 is the method of any of embodiment(s) 1-10, wherein the avexitide is administered by IV infusion at about 0.1 to 1.0 mg/kg/hr, or at about 0.2 to 0.6 mg/kg/hr.

Embodiment 12 is the method of any of embodiment(s) 2-11, wherein the avexitide is administered at a total daily dose of about 60 or about 120 mg.

Embodiment 13 is the method of any of embodiment(s) 3-11, wherein the avexitide is administered once daily (QD) or twice daily (BID).

Embodiment 14 is the method of any of embodiment(s) 1-6, 9-11, and/or 13, wherein the subject is administered avexitide at about 30 mg to about 90 mg BID, and wherein the subject is not required to fast after dosing or is not required to delay a first meal of the day.

Embodiment 15 is the method of any of embodiment(s) 1-6, 8, 10, 11, and/or 13, wherein the avexitide is administered at 45 mg - 120 mg QD, and wherein the subject is required to fast after dosing or to delay the first meal of the day.

Embodiment 16 is the method of embodiment(s) 15, wherein the delay is from between about 30 minutes to about 1.5 hours.

Embodiment 17 is the method of embodiment(s) 15 and/or 16, wherein the subject is required to fast overnight before a morning dose.

Embodiment 18 is the method of embodiment(s) 15-17, wherein the subject is required to fast for about 45 - 90 minutes after a morning dose.

Embodiment 19 is the method of any of embodiment(s) 15-18, wherein the subject is required to fast overnight before a morning dose and for approximately 45 - 90 minutes after the morning dose.

Embodiment 20 is the method of any of embodiment(s) 1-6, wherein the avexitide is administered at a dose of 30 mg BID or 45 mg BID.

Embodiment 21 is the method of embodiment(s) 20, wherein the method comprises administering a morning dose and an evening dose, and wherein the evening dose is administered about 12 hours after the morning dose.

Embodiment 22 is the method of embodiment(s) 21, wherein the morning dose is administered at least about 30 minutes to about 60 minutes before a meal.

Embodiment 23 is the method of any of embodiment(s) 1-6, wherein the avexitide is administered at a dose of 60 mg QD.
Embodiment 24 is the method of embodiment(s) 23, wherein the avexitide is administered in the morning.

Embodiment 25 is the method of embodiment(s) 23 and/or 24, wherein the avexitide is administered at least about 30 minutes to about 90 minutes before a first meal of a day.

Embodiment 26 is the method of any of embodiment(s) 1-25, wherein the formulation comprises avexitide at a concentration from 30 mg/mL to 180 mg/mL; or from 2 mg/mL to 29 mg/mL.

Embodiment 27 is the method of any of embodiment(s) 1 to 25, wherein the formulation comprises avexitide at a concentration from between 45 mg/ml and 90 mg/ml.

Embodiment 28 is the method of embodiment(s) 26, wherein the formulation comprises avexitide at a concentration of 30 mg/mL.

Embodiment 29 is the method of embodiment(s) 26, wherein the formulation comprises avexitide at a concentration of 90 mg/mL.

Embodiment 30 is the method of any of embodiment(s) 1 to 29, wherein the formulation has a pH of about 5.5.

Embodiment 31 is the method of any of embodiment(s) 1 to 30, wherein the formulation comprises a sodium acetate buffer.

Embodiment 32 is the method of any of embodiment(s) 1 to 31, wherein the formulation comprises a tonicity modifier.

Embodiment 33 is the method of embodiment(s) 32, wherein the tonicity modifier comprises mannitol.

Embodiment 34 is the method of any of embodiment(s) 1-33, wherein avexitide is administered for 30 days or less or at least 30 days.

Embodiment 35 is the method of any of embodiment(s) 1-34, wherein treatment reduces the number and/or severity of postprandial neuroglycopenic symptoms in the subject.

Embodiment 36 is the method of any of embodiment(s) 1-35, wherein treatment improves postprandial glucose nadir for the subject.

Embodiment 37 is the method of any of embodiment(s) 1-36, wherein treatment reduces postprandial insulin peak for the subject.

Embodiment 38 is the method of any of embodiment(s) 1-37, wherein treatment reduces the rate of hypoglycemia in the subject.

Embodiment 39 is the method of embodiment(s) 1, wherein the administration reduces the number and/or severity of postprandial neuroglycopenic symptoms in the subject.

Embodiment 40 is the method of any of embodiment(s) 1-6, wherein the administration improves postprandial glucose nadir for the subject.

Embodiment 41 is the method of embodiment(s) 40, wherein there is a significant increase the mixed meal tolerance test ("MMTT") glucose nadir for the subject.

Embodiment 42 is the method of embodiment(s) 41, wherein the mean plasma glucose nadir during MMTT provocation increase by 21% - 25% for the subject compared to a control.

Embodiment 43 is the method of embodiment(s) 40, wherein the subject requires fewer glycemic rescues after treatment as compared to a control.

Embodiment 44 is the method of embodiment(s) 39, wherein peak postprandial glucose values in the subject increase, and wherein this corresponds to a significantly accelerated time to glucose peak during avexitide administration.

Embodiment 45 is the method of any of embodiment(s) 1-44, wherein the administration reduces postprandial insulin peak in the subject.

Embodiment 46 is the method of embodiment(s) 45, wherein mean postprandial insulin peak during an MMTT provocation is reduced from about 21% to 23% in the subject compared to a control.

Embodiment 47 is the method of any of embodiment(s) 1-46, wherein peak GLP-1 and glucagon levels are higher in the subject as compared to a control.

Embodiment 48 is the method of any of embodiment(s) 1-47, wherein GLP-1 and glucagon AUC values are higher during the MMTT in the subject as compared to a control.

Embodiment 49 is the method of any of embodiment(s) 1-48, wherein the subject experiences less frequent hypoglycemic episodes during avexitide treatment as compared to a control.

Embodiment 50 is the method of any of embodiment(s) 1-49, wherein the administration reduces the rate of hypoglycemia in the subject.

Embodiment 51 is the method of any of embodiment(s) 1-50, wherein the administration reduces the rate of severe hypoglycemia in the subject.

Embodiment 52 is the method of any of embodiment(s) 1-51, wherein the administration reduces severe hypoglycemia in the subject.

Embodiment 53 is the method of embodiment(s) 52, wherein severe hypoglycemia comprises symptoms requiring the subject to receive third party assistance.

Embodiment 54 is the method of any of embodiment(s) 1-53, wherein the administration reduces the rate of hypoglycemia-induced CNS impairment in the subject.

Embodiment 55 is the method of any of embodiment(s) 1-54, wherein the administration provides clinically meaningful improvement in the subject in one or more of the rate of hypoglycemia induced CNS impairment, severe hypoglycemia, postprandial insulin peak, rate of severe hypoglycemia, or postprandial glucose nadir.

Embodiment 56 is the method of any of embodiment(s) 1-55, wherein the administration results in an improvement in postprandial hypoglycemia in the subject.

Embodiment 57 is the method of any of embodiment(s) 1-56, wherein the administration results in an increase in postprandial plasma glucose nadir in the subject as compared to baseline or a control.

Embodiment 58 is the method of any of embodiment(s) 1-58, wherein the administration results in an improvement in postprandial neuroglycopenic symptoms or hypoglycemic symptoms in the subject.

Embodiment 59 is the method of any of embodiment(s) 1-59, wherein the avexitide is administered twice daily (BID) within about 60 minutes prior to morning and evening meals, or prior to two main meals of the day for the subject.

Embodiment 60 is the method of any of embodiment(s) 1-60, wherein the avexitide is administered BID and at least about 6 hours apart.

Embodiment 61 is the method of any of embodiment(s) 1-61, wherein the avexitide is administered in a total volume ranging from 0.25-2.0 ml; or in a total volume ranging from about 0.05-0.1 ml; or in an injection volume ranging from 0.25-1.5 ml, or from 0.5-1 ml, or from 0.7-1 ml, or from 0.05-0.1 ml.

Embodiment 62 is the method of any of embodiment(s) 1-61, wherein treatment reduces the rate of severe hypoglycemia in the subject.

Embodiment 63 is the method of any of embodiment(s) 1-63, wherein treatment reduces the rate of hypoglycemia-induced CNS impairment in the subject.

Embodiment 64 is the method of embodiment(s) 63, wherein hypoglycemia-induced CNS impairment includes one of more of the following signs or symptoms: cognitive deterioration, abnormal mentation, impaired judgement, personality/behavior change, bizarre behavior, emotional lability, confusion, amnesia, delirium, vision change, blurred vision, double vision, difficulty speaking, slurred speech, nonsensical speech, coordination difficulties, ataxia, focal or general motor deficit, excessive sleepiness (somnolence), stupor, presyncope, loss of consciousness, convulsions, generalized or focal seizures, or coma.

Embodiment 65 is the method of embodiment(s) 63, wherein the signs and symptoms associated with lowest blood glucose concentrations comprise one or more of loss of consciousness (LOC), slurred speech, visual changes (blurred vision, double vision), bizarre behavior, confusion, and coordination difficulties.

Embodiment 66 is the method of any of embodiment(s) 1-65, wherein the subject is refractory to dietary treatment.

Embodiment 67 is the method of any of embodiment(s) 1-66, wherein the avexitide is administered at a dose of 45 mg BID.

Embodiment 68 is the method of any of embodiment(s) 17-19, and 21, wherein the morning dose is administered from between about 30 minutes to about 90 minutes before a meal.

Embodiment 69 is the method of any of embodiment(s) 1-68, wherein treatment reduces the rate of rescue needed by the subject.

Embodiment 70 is the method of embodiment(s) 69, wherein rescue comprises administering glucagon to the subject at the earlier of glucose ≤ 50 mg/dL + neuroglycopenia or glucose ≤ 40 mg/dL +/- neuroglycopenia.

Embodiment 71 is the method of embodiment(s) 69 and/or 70, wherein the subject is experiencing daily or weekly symptoms of hypoglycemia.

Embodiment 72 is the method of any of embodiment(s) 1-71, wherein the percent time in Hypoglycemia by CGM threshold for the subject is reduced after administration.

Embodiment 73 is the method of any of embodiment(s) 1-72, wherein the number of episodes of hypoglycemia by CGM threshold for the subject is reduced after administration.

Embodiment 74 is the method of any of embodiment(s) 1-73, wherein there were fewer interruptions in daily activities for the subject as compared to a control.

Embodiment 75 is the method of any of embodiment(s) 1-74, wherein the mean rates of hypoglycemia in the subject are reduced from between about 30% - 60% for avexitide as compared to a control.

Embodiment 76 is the method of any of embodiment(s) 1-75, wherein rates of hypoglycemia are an SBGM glucose level <70 mg/dL.

Embodiment 77 is the method of any of embodiment(s) 1-76, wherein the mean rates of clinically important hypoglycemia in the subject are reduced by between about 40% - 59% as compared to a control.

Embodiment 78 is the method of embodiment(s) 77, wherein the clinically important hypoglycemia are any neuroglycopenic symptoms/signs confirmed by SBGM glucose level <55 mg/dL or as a hypoglycemic episode that required assistance of another person to administer rescue therapy.

Embodiment 79 is the method of any of embodiment(s) 1-78, wherein the rates of rescue with oral or G-tube intake for the subject are substantially reduced.

Embodiment 80 is the method of any of embodiment(s) 1-79, wherein the mean percent time spent in hypoglycemia during daytime hours (8am-midnight) for the subject is substantially reduced as compared to a control.

Embodiment 81 is the method of embodiment(s) 80, wherein the mean percent time with glucose values <70 mg/dL, <55 mg/dL, and <40 mg/dL for the subject is reduced by between about 4 - 57% as compared with a control.

Embodiment 82 is the method of embodiment(s) 80 and/or 81, wherein the mean percent time with glucose values <70 mg/dL, <55 mg/dL, and <40 mg/dL for the subject is reduced by between about 36 - 57% as compared with a control.

Embodiment 83 is the method of embodiment(s) 80 and/or 81, wherein the mean percent time with glucose values <70 mg/dL, <55 mg/dL, and <40 mg/dL for the subject is reduced by between about 4 - 26% as compared with a control

Embodiment 84 is the method of any of embodiment(s) 1-83, wherein the mean number of hypoglycemic episodes sustained for at least 10 minutes during daytime hours (8am-midnight) for the subject is substantially reduced as compared to a control.

Embodiment 85 is the method of embodiment(s) 84, wherein the mean number of hypoglycemic episodes (CGM values sustained for at least 10 minutes within a 3-hour period) <70 mg/dL, <55 mg/dL, and <40 mg/dL for the subject is reduced by about 28% - 53% or by about 23% - 29% as compared to a control.

Embodiment 86 is the method of any of embodiment(s) 42, 43, 46-49, 57, 74, 75, 77, and 81-86, wherein the control comprises a comparison to the subject's postprandial plasma glucose nadir prior to the onset of treatment or as measured during MMTT.

Embodiment 87 is the method of any of embodiment(s) 42, 43, 46-49, 57, 74, 75, 77, and 81-86, wherein the control comprises a comparison to a control subject that is not administered the buffered liquid formulation.

### Examples

The following examples are provided to illustrate, but not to limit, the claimed invention.

### EXAMPLE 1: Clinical Study Protocol for Treating Patients With PBH

This example describes a Phase 2 clinical protocol for studying the efficacy, safety, and pharmacodynamic effect of avexitide in patients with post-bariatric hypoglycemia (PBH). In particular, the protocol is a Phase 2, multicenter, randomized, single-blind, placebo-controlled cross-over study to assess the efficacy and safety of avexitide in patients with postbariatric hypoglycemia.

Avexitide was studied for the treatment of hypoglycemia associated with bariatric surgery (e.g., postbariatric hypoglycemia ("PBH")). See Examples infra for further results of the studies outlined in this Example 1.

An efficacy objective was to evaluate the effect of the addition of avexitide to dietary treatment on postprandial hypoglycemia in patients with PBH. It was also an objective to evaluate the safety and tolerability of avexitide. Other objectives included the evaluation of the effect of the addition of avexitide to dietary treatment on postprandial neuroglycopenic signs and symptoms; the evaluation of the effect of the addition of avexitide to dietary treatment on postprandial hyperinsulinemia; the assessment of the rate of hypoglycemia; the assessment of the rate of severe hypoglycemia; the assessment of the rate of hypoglycemia prevention/rescue with glucagon; the assessment of the rate of hypoglycemia prevention/rescue with oral/gastrostomy tube intake; the assessment of postprandial glycemia including duration of hypoglycemia and duration of hyperglycemia; the assessment of nocturnal glycemia; the assessment of postprandial GLP-1 and glucagon levels; the evaluation of quality of life (QOL)/Performance Status; the assessment of development of anti-exendin antibodies (ADA); and the evaluation of avexitide pharmacokinetic exposure/response relationship.

This was a Phase 2, randomized, single-blind, placebo-controlled cross-over study of avexitide in patients with refractory PBH. Eligible participants had a confirmed diagnosis of PBH and at least 2 episodes of severe hypoglycemia during screening in 2-week Run-In Phase: hypoglycemic symptoms confirmed by self blood glucose monitoring (SBGM) glucose ≤ 54 mg/dL (IHSG 2017) (if SBGM is not obtained at the time of symptoms, severe hypoglycemia may be documented based on a corresponding continuous glucose monitor (CGM) measure ≤ 54 and/or neurological recovery following the return of glucose to normal). Additionally, a hypoglycemic episode that requires assistance of another person to administer rescue therapy was considered severe hypoglycemia (Seaquist 2013).

Participants were randomized and assigned in a 1:1 ratio to initially receive one of the following doses self-administered via subcutaneous (SC) injection: (1) placebo followed by avexitide, 30 mg q12 hrs, or (2) placebo followed by avexitide, 60 mg qAM.

Following 14 days of active drug treatment, patients taking 30 mg q12 crossed over to 60 mg qAM and patients taking 60 mg qAM crossed over to 30 mg q12.

On Days 15, 29, and 43, participants underwent in-clinic mixed meal tolerance test (MMTT) provocation with concomitant blood draws and symptom assessments.

Patients underwent Screening within 4 weeks of randomization and enrollment, during which eligibility was confirmed based on the frequency of hypoglycemic episodes. Following confirmation of eligibility based on the initial Screening visit, the 2-week Run-in phase could occur anytime within the 28 day Screening period.

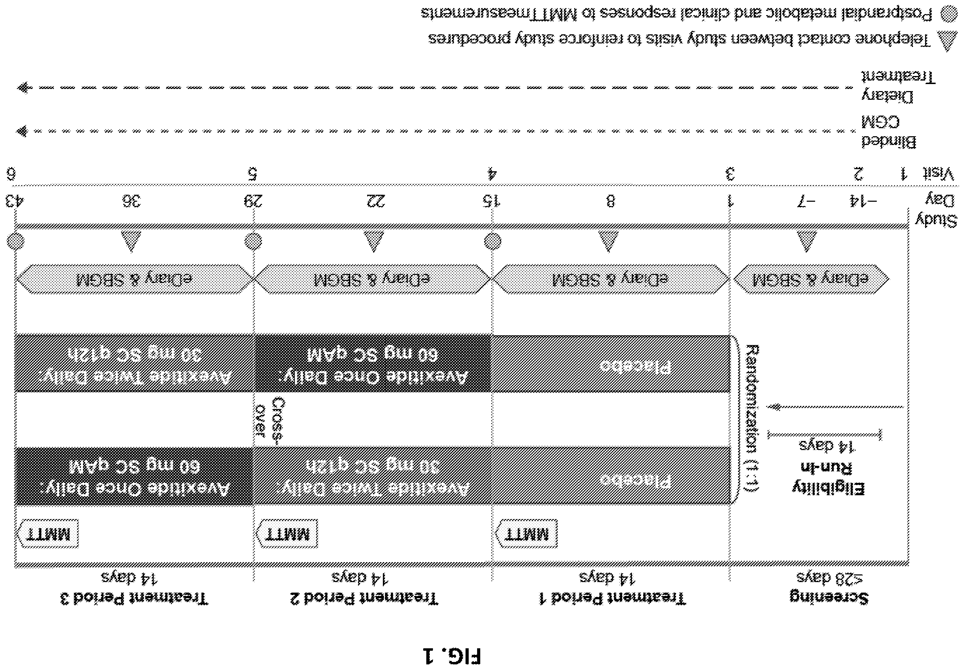

The study consisted of three 14-day treatment periods, for a total of 42 days (see Figure 1). Following randomization, patients administered placebo for 14 days followed by avexitide for 28 days. All patients received placebo and active treatment. Patients will be informed that they will be on placebo for one of the three 14-day treatment periods but will not be informed which one [single-blind]. In addition, patients will receive dietary instruction and will be expected to adhere to currently accepted dietary treatment guidelines for PBH throughout all treatment periods. Patients will return to the study site 30 days after the last dose of study drug for a safety follow-up visit. The maximum anticipated time an individual patient will participate in the study will be ~100 days.

Inclusion criteria included, ability to understand the purpose and risks of the study; willing and able to adhere to scheduled visits, treatment plans, laboratory tests, procedures related to the use of the CGM device, and other study evaluations and procedures, and provide written informed consent; 18-65 years old; a body mass index (BMI) of up to 40 kg/m2; a stable body weight, i.e., not varying by >5% for at least 4 months; a documented Roux-en-Y gastric bypass (RYGB) surgery performed ≥12 months before the start of screening; diagnosis of PBH documented in the medical record and defined as: presence of symptomatic postprandial hypoglycemia associated with inappropriately elevated insulin (≥3 µU/mL) or C-peptide (>0.6 ng/mL) at the time of hypoglycemia (≤54 mg/dL glucose); and at least 2 episodes during screening 2-week Run-in phase of severe hypoglycemia: hypoglycemic symptoms confirmed by SBGM glucose ≤54 mg/dL (IHSG 2017) (if SBGM is not obtained at the time of symptoms, severe hypoglycemia may be documented based on a corresponding CGM measure ≤ 54 and/or neurological recovery following the return of glucose to normal). Additionally, a hypoglycemic episode that requires assistance of another person to administer rescue therapy is considered severe hypoglycemia (Seaquist 2013).

Patients meeting any of the following criteria were excluded from the study:
- Participation in another interventional clinical study within 30 days before Screening.
- Donation or loss of >500 mL of blood or blood product within 56 days before the first dose of study drug.
- Hypoglycemia predating RYGB surgery with the exception of hypoglycemia resulting from prior use of anti-hyperglycemic medications.
- History of or current insulinoma or other cause of endogenous hyperinsulinism other than PBH (e.g., insulin autoimmune hypoglycemia).
- Clinically significant active infection within 14 days before the first dose of study drug.
- History of any clinically relevant acute or chronic psychiatric, renal, hepatic, pancreatic, cardiovascular, neurological, hematological, or gastrointestinal abnormality (e.g., inflammatory bowel disease) or prior malignancy from which patient has been disease free for <5 years with exception of adequately treated basal cell or squamous cell skin cancer, or in situ cervical cancer.
- Major surgery within 6 months before randomization.
- History of other metabolic or bariatric surgical procedure (e.g., vertical sleeve gastrectomy, gastric band).
- History of non-RYGB upper GI surgery (e.g., esophagectomy, gastrectomy, gastrostomy tube). Abnormal liver function, defined as transaminases (alanine transaminase [ALT], aspartate transaminase [AST]) levels >2 × upper limit of normal (ULN) and/or bilirubin level >2 × ULN at Screening.
- Abnormal renal function, defined as a glomerular filtration rate (GFR) <60 mL/min/1.73 m2 (calculated using the Chronic Kidney Disease Epidemiology Collaboration (CKD-EPI) equation) at Screening.
- Abnormal electrolyte concentrations including potassium, magnesium, phosphorus and sodium.
- Use of agents that may interfere with glucose metabolism within 5 half-lives, as follows:
   ∘ Anti-hyperglycemic agents (eg, insulin, sulfonylureas, meglitinides, metformin, thiazolidinediones [TZDs], dipeptidyl-peptidase [DPP-4] inhibitors, sodium-glucose-linked transporter 2 [SGLT2] inhibitors, GLP-1 agonists);
   ∘ Alcohol (e.g., beer, wine, drinks containing distilled spirits);
   ∘ Pentamidine;
   ∘ Lithium;
   ∘ Fluoroquinolone antibiotics (eg, ciprofloxacin, gemifloxacin, levofloxacin, moxifloxacin, norfloxacin, ofloxacin);
   ∘ Tramadol (chronic use of other opioids is permitted as long as dose remains stable throughout study);
   ∘ Current use of acarbose, verapamil, diazoxide and or somatostatin analogs (eg. octreotide, pasirotide, lanreotide)
   ∘ Systemic or inhaled glucocorticoids (e.g., prednisone, prednisolone, methylprednisolone, beclomethasone, betamethasone, dexamethasone, hydrocortisone, triamcinolone) with exception of topically or nasally administered steroids; or
   ∘ Acetaminophen.

Avexitide Injection, 30 mg/mL is a sterile, clear, colorless solution and is supplied in single-use 2 mL vials containing 1 mL solution. The drug product composition consists of 30 mg/mL avexitide (active ingredient) in a sodium acetate buffer containing mannitol as a tonicity modifier. A total of 1 mL solution can be withdrawn from the vial for subcutaneous (SC) injection.

Avexitide Injection is administered subcutaneously in the anterolateral aspect of the thigh.

If the patient exhibits neuroglycopenic symptoms and has a blood glucose ≤ 50 mg/dL, then a final glucose/hormonal blood sample is drawn, and glycemic rescue measures are taken to raise the blood glucose. If the patient remains asymptomatic, blood draws continue until the earlier of (1) the patient develops neuroglycopenic symptoms or (2) the blood glucose falls to 40 mg/dL, at which time a final glucose/hormonal blood sample is drawn, and measures are taken to raise blood glucose irrespective of the presence of symptoms.

Patients will remain fasting during the MMTT until either the 180 minute time point or the time of rescue, if sooner, after which the patient may eat a low carbohydrate snack/meal.

### Efficacy Endpoints

An efficacy endpoint is the magnitude of postprandial hypoglycemia, defined as the plasma glucose nadir occurring within 3 hours of MMTT provocation. The plasma glucose nadir obtained after 14 days of avexitide plus dietary treatment (Treatment Periods 2 and 3) will be compared to after 14 days of placebo administration plus dietary treatment (Treatment Period 1). Particularly, the placebo-adjusted plasma glucose nadir will be calculated for each patient and evaluated.

Inter-subject and intra-subject variability will be derived for the primary efficacy endpoint.

### Safety Endpoints

The primary safety endpoints include evaluation of adverse events (e.g., incidence of treatment-emergent adverse events [TEAEs], serious adverse events [SAEs], TEAEs leading to discontinuation) and other safety evaluation (e.g., clinical laboratory results, vital sign measurements).

### Other Endpoints

### Postprandial Neuroglycopenic Signs and Symptoms

The severity of neuroglycopenic symptoms in response to meal provocation by MMTT will be evaluated based on a modified version of the EHSS questionnaire (MEHSS), which contains severity-graded EHSS with 11-point numeric rating scale (NRS) (0 = not present, 10 = worst possible). Neuroglycopenic symptoms are those defined by the EHSS (Hepburn 1991, Deary 1993).

The MEHSS questionnaire will be completed by patients approximately every 30 minutes during each 180-minute MMTT (or immediately prior to rescue therapy administration, if prior to 180 min) on Days 15 (Treatment Period 1), 29 (Treatment Period 2) and 43 (Treatment Period 3). The highest severity-graded score recorded for each symptom between the observed peak plasma glucose and the end of the MMTT (or before rescue) will be summed to generate the total score, which will be used to rate the presence and severity of acute neuroglycopenic symptoms in each treatment period.

### Peak Postprandial Insulin Response

The placebo-adjusted peak postprandial insulin concentration in response to meal provocation by MMTT after 14 days of active treatment plus dietary treatment (Treatment Periods 2 and 3) will be calculated and evaluated.

### Requirement for Glycemic Rescue During MMTT

### Rate of hypoglycemia

Any hypoglycemic symptoms confirmed by SBGM glucose <70 mg/dL; or if SBGM is not obtained at the time of symptoms, hypoglycemia may be confirmed based on a corresponding CGM measure <70 mg/dL.

The rate is calculated as number of distinct hypoglycemia episodes divided by number of days for a given treatment period, then normalized to duration of 2 weeks if the treatment period is not exactly 14 days.

### Rate of severe hypoglycemia

Any neuroglycopenic symptoms/signs confirmed by SBGM glucose ≤54 mg/dL (IHSG 2017); if SBGM is not obtained at the time of neuroglycopenic symptoms, severe hypoglycemia may be documented based on a corresponding CGM measure ≤ 54 and/or neurological recovery following the return of glucose to normal. Additionally, a hypoglycemic episode that requires assistance of another person to administer rescue therapy is considered severe hypoglycemia (Seaquist 2013).

Similarly, the rate is calculated as total number of distinct severe hypoglycemia episodes divided by number of days for a given treatment period, then normalized to duration of 2 weeks if the treatment period is not exactly 14 days.

### Percent of time with postprandial glucose measures < 70 mg/dL as measured by CGM

The percent of time is calculated as total number of CGM data points that are <70 mg/dL divided by total number of CGM data points obtained for a given treatment period.

### Percent of time with postprandial glucose measures ≤54 mg/dL as measured by CGM

The percent of time is calculated as total number of CGM data points that are ≤54 mg/dL divided by total number of CGM data points obtained for a given treatment period.

### Percent of time with postprandial glucose measures ≤40 mg/dL as measured by CGM

The percent of time is calculated as total number of CGM data points that are ≤40 mg/dL divided by total number of CGM data points obtained for a given treatment period.

### Mean weighted glucose (MWG) between 1 and 3 hours, 1 and 2 hours, and 2 and 3 hours after meals as measured by CGM

The MWG1-3 hr, MWG1-2 hr, and MWG2-3 hr will be calculated as the total area under the concentration-time curve of glucose levels collected by CGM in the corresponding time period, divided by the actual time span in hours. The linear up/down trapezoidal method will be used for area under the curve calculation.
- Mean amplitude of glycemic excursion (MAGE) between 1 and 3 hours after meals as measured by CGM
- The MAGE is defined as the mean of the difference from nadir to peak for those differences that exceed the standard deviation of the CGM assessments over 3 hours. It will be calculated using the method described in the original paper (Service 1970).
- Percent of time with glucose measures >180 mg/dL and
   o 250 mg/dL as measured by CGM
- The percent of time is calculated as total number of CGM data points that are within the range of > 180 mg/dL and
   ∘ 250 mg/dL, divided by total number of CGM data points obtained for a given treatment period.
- Percent of time with glucose measures >250 mg/dL as measured by CGM
- The percent of time is calculated as total number of CGM data points that are >250 mg/dL, divided by total number of CGM data points obtained for a given treatment period.
- Percent of time with glucose measures <70 mg/dL between the hours of midnight and 6AM as measured by CGM
- The percent of time is calculated as total number of CGM data points collected between 12AM and 6AM that are <70 mg/dL, divided by total number of CGM data points obtained between 12AM and 6AM for a given treatment period.
- Percent of time with glucose measures ≤54 mg/dL between the hours of midnight and 6AM as measured by CGM
- The percent of time is calculated as total number of CGM data points collected between 12AM and 6AM that are ≤54 mg/dL, divided by total number of CGM data points obtained between 12AM and 6AM for a given treatment period.
- Percent of time with glucose measures ≤40 mg/dL between the hours of midnight and 6AM as measured by CGM
- The percent of time is calculated as total number of CGM data points collected between 12AM and 6AM that are ≤40 mg/dL, divided by total number of CGM data points obtained between 12AM and 6AM for a given treatment period.
- Rate of hypoglycemia prevention/rescue with glucagon
- The rate is calculated as number of distinct hypoglycemia prevention/rescue episodes divided by number of days for a given treatment period, then normalized to duration of 2 weeks if the treatment period is not exactly 14 days
- Rate of hypoglycemia prevention/rescue with oral/G-tube intake

The rate is calculated as number of distinct hypoglycemia prevention/rescue episodes divided by number of days for a given treatment period, then normalized to duration of 2 weeks if the treatment period is not exactly 14 days. The distinct hypoglycemia prevention/rescue episodes can be due to, for example, one or more of the following:
- Postprandial GLP-1 and glucagon levels during the MMTT
- QOL as measured by NSS, NOS, HSIS, HIS, and SF-36v2
- Karnofsky Performance Status
- Development of anti-exendin antibodies (ADA)
- Avexitide pharmacokinetic exposure/response relationship

Outcomes include, but are not limited to, improvement in plasma glucose nadir levels, reduction in peak insulin concentrations, and the requirement for rescue during MMTT provocation. In some embodiments, a patient exhibits an improvement in postprandial hypoglycemia, e.g., an increase in postprandial plasma glucose nadir as compared to baseline. In some embodiments, a patient exhibits an improvement in postprandial neuroglycopenic symptoms, e.g., a reduction in the number and/or severity of postprandial neuroglycopenic symptoms as compared to baseline. In some embodiments, a patient exhibits an improvement in postprandial insulin response, e.g., a decrease in peak postprandial insulin concentration as compared to baseline.

### EXAMPLE 2: PREVENT Phase 2 Clinical Trial for Treating Patients With PBH

This example describes results from a Phase 2 clinical trial (PREVENT) assessing the efficacy and safety of 28-day dosing of avexitide in patients with PBH, conducted according to the protocol disclosed in Example 1 above.

Eighteen patients with refractory, severe PBH were enrolled across five academic centers and dosed as outpatients in the PREVENT study. All patients received placebo subcutaneous (SC) injections for 14 days in a single-blinded manner followed by avexitide 30 mg SC formulation (as described above) twice daily (BID) injections for 14 days and 60 mg once daily (QD) injections for 14 days, for a total of 28 days active dosing, in a double-blinded to dose, cross-over design.

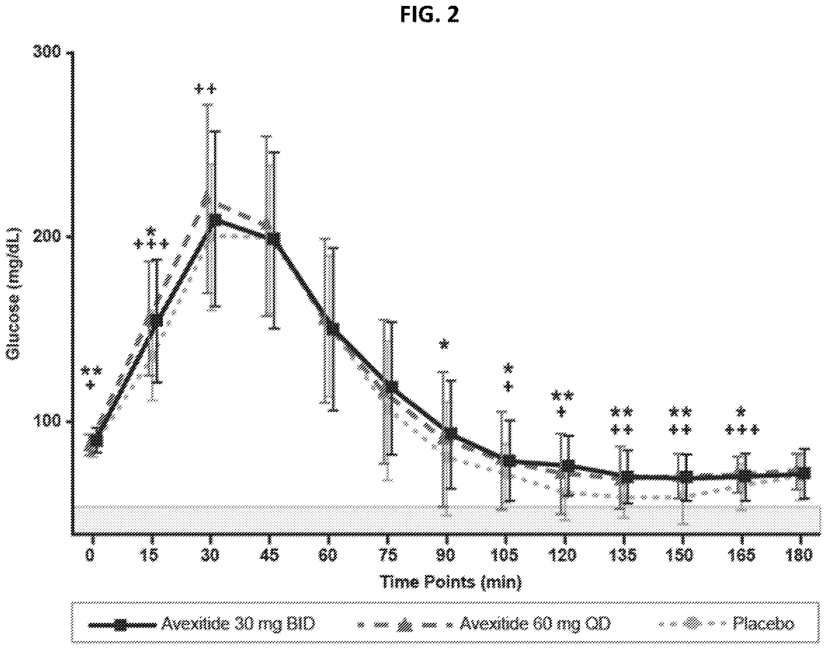

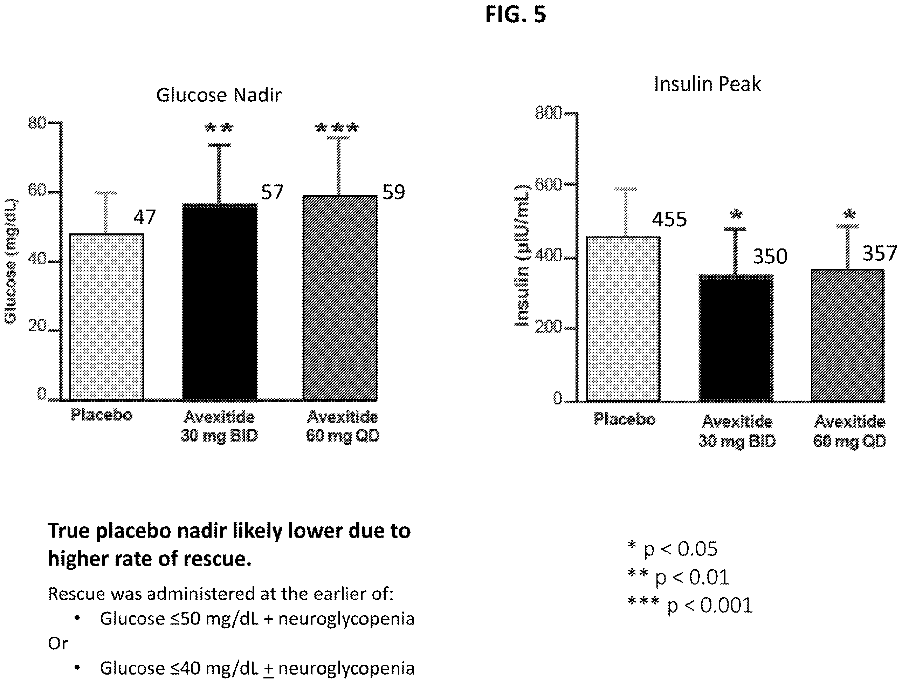

As shown in Table 1 below and in FIG. 5, the primary efficacy endpoint of improved postprandial glucose nadir during mixed meal tolerance testing (MMTT) was achieved with statistical significance with avexitide 30 mg BID (57.1 vs 47.1 mg/dL; p = 0.001) and 60 mg QD (59.2 vs 47.1 mg/dL; p = 0.0002), with fewer participants requiring glycemic rescue during each of the active dosing regimens than during placebo dosing. Glucose measurements for each treatment group were taken at specific time points during MMTT as described in Example 1; mean glucose levels at the different time points are shown in FIG. 2.

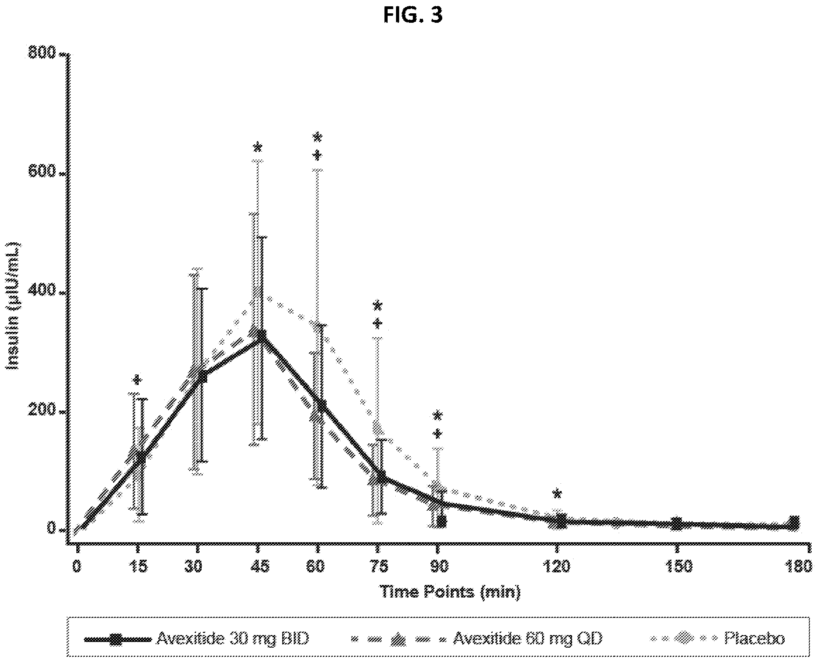

An endpoint of reduced postprandial insulin peak during MMTT was also statistically significant with avexitide 30 mg BID (349.5 vs 454.5 µIU/mL; p < 0.03) and 60 mg QD (357.2 vs 454.5 µIU/mL; p = 0.04) (see, Table 1 below and FIG. 5). Insulin measurements for each treatment group were taken at specific time points during MMTT as described in Example 1; mean insulin levels at the different time points are shown in FIG. 3.

**Table 1. Postprandial glucose nadir and postprandial insulin peak during MMTT**

| | Placebo | 30 mg BID | 60 mg QD | Pval 30 mg BID | Pval 60 mg QD |
|---|---|---|---|---|---|
| Glucose Nadir (mg/dL) | 47.1 | 57.1 | 59.2 | 0.0011 | 0.0002 |
| Insulin Peak (uIU/mL) | 454.5 | 349.5 | 357.2 | 0.0288 | 0.0417 |
| Rescue Administered (%) | 6 (35.3) | 4 (23.5) | 2 (11.8) | | |

The metabolic and clinical status of patients in the outpatient setting throughout all treatment periods was assessed through the use of electronic diaries (eDiary) and continuous glucose monitoring (CGM). Participants were trained by site personnel on the use of an eDiary at the start of the Screening Run-in period (Visit 2a) and maintained the eDiary during the 3 treatment periods. All episodes of hypoglycemic symptoms/signs were to be recorded in the eDiary along with the following information regarding each hypoglycemic episode: specific symptoms/signs experienced; self-blood glucose monitor (SBGM) reading; action taken to treat or prevent the episode (oral intake, glucagon, etc.); and whether assistance from another individual was required; whether they ate or drank something within 3 hours of the episode. The rate of hypoglycemia was assessed and defined as any hypoglycemic symptoms confirmed by SBGM glucose <70 mg/dL. The rate was calculated as the number of distinct hypoglycemia episodes divided by number of days for a given treatment period, then normalized to duration of 2 weeks if the treatment period is not exactly 14 days. The rate of severe hypoglycemia was assessed and defined as any neuroglycopenic symptoms/signs confirmed by SBGM glucose <55 mg/dL (IHSG 2017). Additionally, a hypoglycemic episode that required the assistance of another person to administer rescue therapy was considered severe hypoglycemia (Seaquist 2013). Similarly, the rate was calculated as total number of distinct severe hypoglycemia episodes divided by number of days for a given treatment period, then normalized to duration of 2 weeks if the treatment period is not exactly 14 days.

Metabolic and clinical improvements were observed during avexitide dosing as compared to during placebo dosing, as demonstrated by the reduction in the rates of hypoglycemia, severe hypoglycemia, and rescue (Tables 3-5). Rate of rescue as used herein is the number of distinct hypoglycemia prevention/rescue episodes, including those provided by third party assistance, divided by number of days for a given treatment period, then normalized to duration of 2 weeks if the treatment period was not exactly 14 days. Patients experienced fewer episodes of hypoglycemia (hypoglycemia symptoms confirmed by self-blood glucose monitor (SBGM) concentrations of <70 mg/dL) and severe hypoglycemia (neuroglycopenic symptoms confirmed by SBGM concentrations <55 mg/dL) during both dosing regimens of avexitide as compared to placebo (Table 2). These results were corroborated by CGM data (Table 3). As shown in Table 4, the placebo-adjusted rate of severe hypoglycemia was significantly reduced during avexitide 30 mg BID dosing (-0.89 +/-0.36 episodes/14 days, p=0.0267) and 60 mg QD dosing (-1.35 +/- 0.36 episodes/14 days, p=0.0020). In addition, episodes of hypoglycemia and severe hypoglycemia that were confirmed as postprandial in nature, as defined as occurring within 3 hours of having eaten, were evaluated. The placebo-adjusted rate of postprandial hypoglycemia and severe hypoglycemia were significantly lower during both avexitide dosing regimens as compared to placebo. In particular, the placebo-adjusted rate of severe postprandial hypoglycemia was significantly reduced during avexitide 30 mg BID dosing (-0.94 episodes/14 days, p=0.0172), and 60 mg QD dosing (-1.2 episodes/14 days, p=0.0038) (Table 4). As shown in Table 4, the rates of hypoglycemia rescue based on eDiary recordings were substantially reduced during avexitide treatment: Mean pooled rate of hypoglycemia rescue by self or third-party during placebo, 4.87; avexitide 30 mg BID, 3.34 (p = 0.0614); and avexitide 60 mg QD 1.83 (p = 0.0013); Mean pooled rate of hypoglycemia rescue by third-party during placebo, 0.63; avexitide 30 mg BID, 0.28 (p = 0.1194); and avexitide 60 mg QD, 0.24 (p = 0.0856). Most of the rescues were self-administered without assistance of a third party. Accordingly, the rates for third-party rescue were low overall. Nevertheless, the results suggest a reduced requirement for third party rescue with avexitide treatment.

As shown in Table 3, these results captured by the patient eDiary were also corroborated by CGM results, which showed reductions in the percent time in hypoglycemia and number of episodes of hypoglycemia during the diurnal period (8am to midnight).

**Table 2. eDiary**

| Reduction in Rates¹ of Hypoglycemia, Severe Hypoglycemia and Rescue as Collected by SBGM + eDiary | | | |
|---|---|---|---|
| | Number of Episodes in 14 Day Period | | |
| | Placebo | 30 mg BID | 60 mg QD |
| Rate of Hypoglycemia² | 4.03 | 2.81 | 1.56 |
| Change from Placebo | NA | -1.24 (p=0.0720) | -2.51 (p=0.0014) |
| Rate of Severe Hypoglycemia³ | 2.36 | 1.45 | 0.99 |
| | NA | -0.89 (p=0.0267) | -1.35 (p=0.0020) |
| Rate of Postprandial Hypoglycemia | 3.46 | 2.24 | 1.27 |
| Rate of Severe Postprandial Hypoglycemia | 1.91 | 0.94 | 0.69 |
| Rate of Rescue⁴ | 4.87 | 3.34 | 1.83 |
| | NA | -1.60 (p=0.0614) | -3.13 (p=0.0013) |
| Rate of Rescue by Third-Party | 0.63 | 0.28 | 0.24 |
| | | -0.34 (p=0.1194) | -0.38 (p=0.0856) |

| | | | |
|---|---|---|---|
| ¹ Rate is defined as number of episodes in a 14-day period ² Hypoglycemia is defined as hypoglycemia symptoms confirmed by SBGM concentrations of <70 mg/dL ³ Severe hypoglycemia is defined as neuroglycopenic symptoms confirmed by SBGM concentrations <55 mg/dL ⁴ Rescue is defined as requiring self- or third-party administration of oral or g-tube intake to prevent or treat hypoglycemia | | | |

**Table 3. CGM**

| | Placebo | 30 mg BID | 60 mg QD |
|---|---|---|---|
| Percent time <70 Diurnal | 7.810 | 4.971 | 5.787 |
| Percent time <55 Diurnal | 2.119 | 1.073 | 1.611 |
| Percent time <40 Diurnal | 0.541 | 0.234 | 0.522 |
| No. Episodes <70 for 10 min Diurnal | 18.8 | 13.0 | 13.8 |
| No. Episodes <55 for 10 min Diurnal | 6.1 | 3.4 | 4.1 |
| No. Episodes <40 for 10 min Diurnal | 1.6 | 0.7 | 1.1 |
| No. Episodes <70 for 30 min Diurnal | 10.8 | 6.2 | 7.8 |
| No. Episodes <55 for 30 min Diurnal | 2.7 | 1.8 | 1.9 |
| No. Episodes <40 for 30 min Diurnal | 0.6 | 0.2 | 0.5 |
| No. Episodes <70 for 60 min Diurnal | 3.4 | 2.2 | 2.2 |
| No. Episodes <55 for 60 min Diurnal | 0.7 | 0.2 | 0.5 |
| No. Episodes <40 for 60 min Diurnal | 0.1 | 0.1 | 0.2 |

**Table 4. Hypoglycemia Rate and Severe Hypoglycemia Rate Collected by ediary (Mixed Model Statistical Analysis)**

| Parameter | Avexitide Treatment | Estimate | Std Error | P value | Lower | Upper |
|---|---|---|---|---|---|---|
| Rate of Hypoglycemia | 30 mg q12 hrs | -1.2444 | 0.6430 | 0.0720 | -2.6149 | 0.1260 |
| Rate of Hypoglycemia | 60 mg qAM | -2.5076 | 0.6430 | 0.0014 | -3.8781 | -1.1372 |
| Rate of Hypoglycemia that Occurred Within 3 Hours of Meal/Snack | 30 mg q12 hrs | -1.2444 | 0.6337 | 0.0684 | -2.5951 | 0.1062 |
| Rate of Hypoglycemia that Occurred Within 3 Hours of Meal/Snack | 60 mg qAM | -2.2521 | 0.6337 | 0.0029 | -3.6027 | -0.9015 |
| Rate of Severe Hypoglycemia | 30 mg q12 hrs | -0.8917 | 0.3629 | 0.0267 | -1.6651 | -0.1182 |
| Rate of Severe Hypoglycemia | 60 mg qAM | -1.3528 | 0.3629 | 0.0020 | -2.1262 | -0.5793 |
| Rate of Severe Hypoglycemia that Occurred Within 3 Hours of Meal/Snack | 30 mg q12 hrs | -0.9417 | 0.3515 | 0.0172 | -1.6908 | -0.1925 |
| Rate of Severe Hypoglycemia that Occurred Within 3 Hours of Meal/Snack | 60 mg qAM | -1.2028 | 0.3515 | 0.0038 | -1.9519 | -0.4536 |

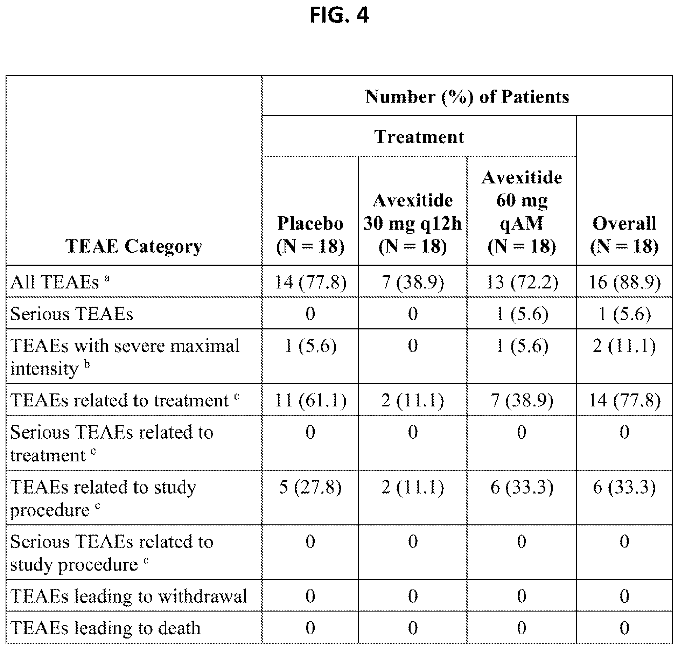

Avexitide was well-tolerated. There were no treatment-released serious adverse events and no participant withdrawals. Adverse events were typically mild to moderate in severity. The most common adverse events were injection site bruising, nausea, and headache, all of which occurred with lower frequency during each of the avexitide dosing periods than during the placebo dosing period (Figure 4).

### EXAMPLE 3: Phase 2 Clinical Trial for Treating Patients With PBH

Table 5 describes the severity of the disorder of the patients enrolled in the Phase 2 clinical trial described above. The study enrolled patients with severe, refractory post-bariatric hypoglycemia. Nearly half of all participants (44.4%) reported a history of loss of consciousness, 11.1% reported a history of seizure, and 16.7% reported a history of hospitalization due to PBH. Ninety-five percent of participants reported daily or weekly symptoms of hypoglycemia. All patients were refractory to dietary intervention and 83% were refractory to medical nutrition therapy, with 3 study participants resorting to surgical interventions for treatment of severe refractory PBH.

**Table 5. Characteristics of Study Subjects**

| Subject Disposition | | Treatment Sequence | | | |
|---|---|---|---|---|---|
| | | Placebo, Avexitide 30 mg q12h, Avexitide 60 mg qAM a (N = 8) | | Placebo, Avexitide 60 mg qAM, Avexitide 30 mg q12h b (N = 10) | Overall (N = 18) |
| Time since RYGB surgery (months) | | | | | |
| | Mean (SD) | 88.1 (43.29) | | 97.8 (63.60) | 93.5 (54.20) |
| | Median | 70.5 | | 89 | 82 |
| | Min, Max | 48, 173 | | 16, 198 | 16, 198 |
| Pre-operative weight (kg) | | | | | |
| | Mean (SD) | 124.739 (29.5588) | | 131.542 (14.2051) | 128.518 (21.8791) |
| | Median | 133.13 | | 133.355 | 133.13 |
| | Min, Max | 52.62, 145.15 | | 108.41, 154.22 | 52.62, 154.22 |
| Duration and time with PBH (time since PBH diagnosis) (months) | | | | | |
| | Mean (SD) | 24.4 (28.91) | | 16.7 (15.54) | 20.1 (22.08) |
| Median | | | 11 | 14.5 | 14.5 |
| Min, Max | | | 0, 69 | 0, 50 | 0, 69 |
| Time from bariatric surgery to the first experience of postprandial hypoglycemia (months) | | | | | |
| | Mean (SD) | | 24.1 (30.69) | 44.9 (53.42) | 35.7 (44.85) |
| | Median | | 12.5 | 17.5 | 14 |
| | Min, Max | | 2, 96 | 6, 144 | 2, 144 |
| Frequency of seizure | | | | | |
| | Monthly | | 0 | 1 (10.0) | 1 (5.6) |
| | Rarely (has occurred once or less than yearly) | | 0 | 1 (10.0) | 1 (5.6) |
| | Never | | 8 (100.0) | 8 (80.0) | 16 (88.9) |
| Frequency of symptoms of hypoglycemia | | | | | |
| | Weekly¹ | | 5 (62.5) | 5 (50.0) | 10 (55.6) |
| | Monthly¹ | | 0 | 1 (10.0) | 1 (5.6) |
| History of Type 2 diabetes before RYGB surgery | | | | | |
| | No | | 8 (100.0) | 10 (100.0) | 18 (100.0) |
| Hospitalization due to hypoglycemia | | | | | |
| | No | 7 (87.5) | 8 (80.0) | | 15 (83.3) |
| | Yes | 1 (12.5) | 2 (20.0) | | 3 (16.7) |
| Treatments attempted to treat PBH | | | | | |
| | Yes | 8 (100.0) | 10 (100.0) | | 18 (100.0) |

| | | | | | |
|---|---|---|---|---|---|
| 95% of the subjects had fewer daily or weekly symptoms of hypoglycemia. | | | | | |

FIG. 5 demonstrates that treatment with avexitide 30 mg BID and 60 mg QD resulted in a significant reduction in hyperinsulinemic hypoglycemia with a reduced requirement for rescue during MMTT provocation. The primary efficacy endpoint of the PREVENT study was met with statistical significance by each of the two evaluated dosing regimens, with higher placebo-corrected mean postprandial plasma glucose nadirs during MMTT provocation. Congruent with these results, fewer subjects required glycemic rescue during treatment with each of the avexitide dosing regimens than during placebo.

A significant reduction in postprandial hyperinsulinemia was also observed with both dosing regimens, as measured as the placebo-adjusted peak postprandial insulin response to MMTT. Hyperinsulinemia as induced by an exaggerated GLP-1-mediated incretin effect in patients with PBH was effectively mitigated by GLP-1 antagonism demonstrates the targeted therapeutic approach of avexitide.

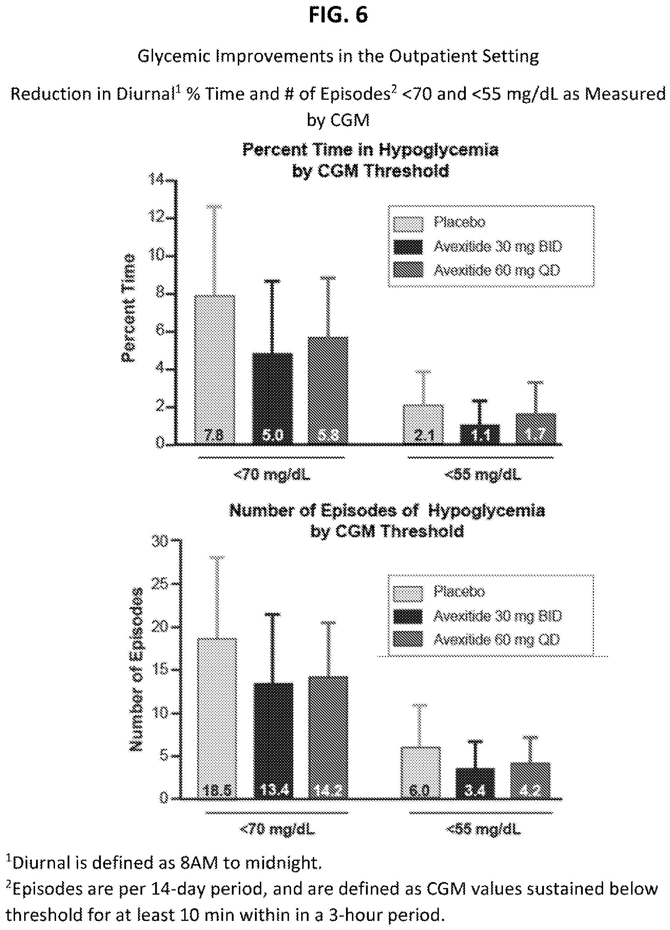

FIG. 6 demonstrates glycemic improvements in the outpatient setting showing that there is a reduction in diurnal percent time and number of episodes <70 and <55 mg/dL as measured by CGM. Diurnal as used herein is 8AM to midnight. Episodes are per 14-day period, and are defined as CGM values sustained below threshold for at least 10 min within in a 3-hour period. These results collected by blinded CGM corroborated both the glycemic benefits observed during in-clinic MMTT assessments, as well as the clinical and metabolic improvements as captured by the patient eDiary, overall demonstrating a reduction in biochemical and symptomatic hypoglycemia during both dosing regimens of avexitide treatment as compared to placebo.

The study demonstrated that there was a low occurrence of development of anti-drug antibodies (ADA). One of 18 participants developed low positive titers for ADA. However, no associated AEs were observed, and there were no differences in measured endpoints for this participant.

The 28-day treatment in outpatient setting demonstrated clinically meaningful improvements including, for example, reductions in the magnitude of postprandial hyperinsulinemic hypoglycemia, reductions in the rates of hypoglycemia and severe hypoglycemia, reductions in the rates of rescue, and reductions in the percent time in hypoglycemia and number of hypoglycemic episodes.

In some instances, 45 mg BID, a higher exposure than 60 mg QD, results in potentially improved daytime coverage, as demonstrated by the more substantial reductions in the rate of hypoglycemia, rate of severe hypoglycemia, and rate of rescue (see Table 3) as compared to the lower (30 BID) regimen.

### EXAMPLE 4

For the study described in Example 2, all patients completed treatments; 17 were included in the efficacy analyses. Compared to placebo, both avexitide regimens significantly increased the MMTT glucose nadir: least squares (LS) mean placebo-corrected glucose nadir was 10.10 mg/dL (95% confidence interval [CI], 4.770 to 15.438, P=0.0011) for avexitide q12h and 12.19 mg/dL (95% CI, 6.854 to 17.521; P=0.0002) for avexitide qAM. The LS mean placebo-corrected postprandial insulin peak was -104.53 µIU/mL (95% CI, -196.689 to -12.380; P=0.0288) for avexitide q12h and -96.29 µIU/mL (95% CI, -188.446 to -4.137; P=0.0417) for avexitide qAM. Consistent with MMTT results, clinically important outpatient hypoglycemic episodes occurred less frequently during avexitide treatment. Avexitide was well-tolerated, with no treatment-related serious adverse events and no withdrawals.

As shown in Table 6, patients were female, relatively young (on average 44.3 years old), mostly white (94.4%) and non-Hispanic (88.9%), with a mean post-RYGB BMI of approximately 30 kg/m2. Patient baseline disease characteristics reflected the severe and refractory nature of PBH in the study population: nearly half of participants reported a history of hypoglycemia-induced loss of consciousness (44.4%), and nearly all (94.4%) reporting either daily or weekly hypoglycemic symptoms. Three patients (16.7%) had been hospitalized due to hypoglycemia; 2 patients (11.1%) reported a history of hypoglycemia-induced seizure of which 1 patient (5.6%) reported monthly seizures.

During the Run-In Period, all eligible patients were confirmed to be refractory to dietary treatment. Most patients (83.3%) had received pharmacological therapy with the off-label use of at least one medication for treatment of PBH, and 3 patients had undergone additional surgical procedures to treat their PBH (placement of gastrostomy tube in 2 patients; RYGB revision in 1 patient).

**Table 6. Participant Baseline Characteristics**

| **Characteristic** | | **Treatment Sequence** | | **Total** |
|---|---|---|---|---|
| | | **Placebo,** | **Placebo,** | **(N = 18)** |
| | | **Avexitide 30 mg q12h,** | **Avexitide 60 mg qAM, Avexitide 30 mg q12h ^{b}** | |
| | | **Avexitide 60 mg qAM ^{a}** | **(N = 10)** | |
| | | **(N = 8)** | | |
| **Demographic/Anthropomorphic Characteristic** | | | | |
| Sex, n female (%) | | 8 (100.0) | 10 (100.0) | 18 (100.0) |
| Age, mean (SD), years | | 45.5 (7.5) | 43.4 (12.0) | 44.3 (10.0) |
| Race, n (%) | | | | |
| | Asian | 1 (12.5) | 0 | 1 (5.6) |
| | White | 7 (87.5) | 10 (100.0) | 17 (94.4) |
| Ethnicity, n (%) | | | | |
| | Hispanic or Latino | 0 | 2 (20.0) | 2 (11.1) |
| | Not Hispanic or Latino | 8 (100.0) | 8 (80.0) | 16 (88.9) |
| Weight, mean (SD), kg | | 81.6 (7.3) | 81.0 (16.1) | 81.23 (12.6) |
| BMI, mean (SD), kg/m² | | 30.0 (3.1) | 29.3 (4.9) | 29.6 (4.1) |

| **Clinical Baseline/History** | | | | |
|---|---|---|---|---|
| Time since RYGB, mean (SD), months | | 88.1 (43.3) | 97.8 (63.6) | 93.5 (54.2) |
| Pre-RYGB weight, mean (SD), kg | | 124.7 (29.6) | 131.5 (14.2) | 128.5 (21.9) |
| Time from RYGB to first experience of postprandial hypoglycemia, mean (SD), months | | 24.1 (30.7) | 44.9 (53.4) | 35.7 (44.9) |
| History of LOC due to PBH, n (%) | | 3 (37.5) | 5 (50.0) | 8 (44.4) |
| History of seizure due to PBH, n (%) | | 0 | 2 (20.0) | 2 (11.1) |
| History of hospitalization due to PBH, n (%) | | 1 (12.5) | 2 (20.0) | 3 (16.7) |
| Frequency of symptoms of hypoglycemia | | | | |
| | Daily, n (%) | 3 (37.5) | 4 (40.0) | 7 (38.9) |
| | Weekly, n (%) | 5 (62.5) | 5 (50.0) | 10 (55.6) |
| | Monthly, n (%) | 0 | 1 (10.0) | 1 (5.6) |
| History of Type 2 diabetes before RYGB surgery, n (%) | | 0 | 0 | 0 |
| Following medical nutrition therapy, n (%) | | 8 (100) | 10 (100) | 18 (100) |
| History of pharmacotherapy for PBH, n (%) | | 5 (63.0) | 10 (100.0) | 15 (83.0) |
| History of surgery for PBH, n (%) | | 1 (5.6) | 2 (11.1) | 3 (16.7) |

| | | | | |
|---|---|---|---|---|
| ^{a} Placebo (Treatment Period 1) then avexitide 30 mg q12h (Treatment Period 2) then avexitide 60 mg qAM (Treatment Period 3). ^{b} Placebo (Treatment Period 1) then avexitide 60 mg qAM (Treatment Period 2) then avexitide 30 mg q12h (Treatment Period 3). | | | | |

As shown in Table 7 and Figure 5, the mean plasma glucose nadir during MMTT provocation was raised by over 21% (57 mg/dL vs 47 mg/dL) and 25% (59 mg/dL vs 47 mg/dL) during avexitide 30 mg q12h and avexitide 60 mg qAM treatments, respectively, compared to during placebo treatment, corresponding to fewer participants requiring glycemic rescue during avexitide versus placebo MMTT provocation. The true placebo nadir is likely lower than the measured nadir (47 mg/dL) due to a higher rate of glycemic rescue. In spite of the higher rate of rescue during placebo MMTT, the LS mean placebo-corrected postprandial plasma glucose nadir during MMTT provocation demonstrated statistically significant increases in glucose nadir for both avexitide 30 mg q12h (LS mean, 10.10 mg/dL; 95% CI, 4.770 to 15.438; P=0.0011) and avexitide 60 mg qAM (LS mean, 12.19 mg/ dL; 95% CI, 6.854 to 17.521; P=0.0002). Thus, the primary endpoint was met. Neither treatment sequence nor treatment period had a statistically significant effect (P>0.2 and P>0.8, respectively). The LS mean difference between the avexitide 60 mg qAM vs avexitide 30 mg q12h vs treatments was small (2.08, 95% CI -3.238 to 7.405) and not statistically significant.

As shown in Table 7, additional glycemic parameters yielded complementary results and were consistent with the mechanism of action of avexitide. Fasting plasma glucose concentrations remained within the normal range during all treatment periods, while peak postprandial glucose values increased during avexitide 60 mg qAM but not avexitide 30 mg q12h treatment, corresponding to a significantly accelerated time to glucose peak during avexitide 60 mg qAM treatment. AUC₀₋₁₈₀ₘᵢₙ glucose increased significantly during both avexitide treatments, and was primarily attributable to an increase in late plasma glucose concentrations during avexitide treatments versus placebo treatment.

As shown in Table 7 and FIGs. 3 and 5, the mean postprandial insulin peak during MMTT provocation was reduced by more than 23% (349.5 µIU/mL vs 454.5 µIU/mL) and 21% (357.2 µIU/mL vs 454.5 µIU/mL) during avexitide 30 mg q12h and avexitide 60 mg qAM treatments, respectively, compared to during placebo treatment. The LS mean placebo-corrected postprandial insulin peak during MMTT provocation showed statistically significant decreases in insulin peak with avexitide 30 mg q12h (LS mean = -104.53 µIU/mL, P= 0.0288) and avexitide 60 mg qAM (LS mean = -96.29 µIU/mL, P= 0.0417). Neither treatment sequence nor treatment period had a statistically significant effect (P>0.7 and >0.6, respectively). The LS mean difference between the avexitide 30 mg q12h vs avexitide 60 mg qAM treatments was small (8.24) and not statistically significant.

### GLP-1 and Glucagon

Fasting GLP-1 and glucagon levels were similar across the avexitide 30 mg q12h and 60 mg qAM treatments compared to placebo treatment. Peak GLP-1 and glucagon levels were higher in the avexitide groups, as were the GLP-1 and glucagon AUC values during the MMTT (Table 7).

### Neuroglycopenic Symptoms

**Table 7. Metabolic and Clinical Responses to Mixed Meal Tolerance Test by Treatment Regimen**

| **Parameter** | **Mean Value (SD)** | | | **Avexitide 30 q12h** | | | **Avexitide 60 qAM** | | |
|---|---|---|---|---|---|---|---|---|---|
| | **(N=17)** | | | **(N = 17)** | | | **(N = 17)** | | |
| | **Placebo** | **Avexitide 30 q12h** | **Avexitide 60 qAM** | **LS Mean Placebo-Corrected Value (SE)** | **95% CI** | **P-value** | **LS Mean Placebo-Corrected Value (SE)** | **95% CI** | **P-value** |
| | **(N = 17)** | **(N =** 17) | **(N =** 17) | | | | | | |
| **Glucose** | | | | | | | | | |
| Fasting (mg/dL) | 83.8 (5.3) | 87.5 (6) | 84.6 (4.2) | 3.8 (1.1) | 1.52, 5.99 | **0.003** | 0.85 (1.05) | -1.38, 3.09 | 0.428 |
| Peak (mg/dL) | 211.8 (38.5) | 218.2 (45.2) | 224.5 (47.3) | 6.3 (4.8) | -3.85, 16.49 | 0.205 | 11.87 (4.77) | 1.70, 22.05 | **0.025** |
| Time to peak (min) | 39.9 (7.4) | 37.9 -8.3 | 34.4 -6.7 | -2 (1.9) | -5.99, 1.92 | 0.29 | -5.72 (1.86) | -9.68, -1.76 | **0.008** |
| Nadir (mg/dL) | 47.1 (127) | 57.1 (16.5) | 59.2 (16.1) | 10.1 (2.5) | 4.77, 15.44 | **0.001** | 12.19 (2.50) | 6.85, 17.52 | **0.000** |
| Peak to nadir difference (mg/dL) | -164.6 (358) | -161.1 (40.1) | -165.4 (43.8) | 3.8 (5.7) | -8.45, 16.02 | 0.52 | 0.31 (5.74) | -11.92, 12.55 | 0.957 |
| Rate of glucose decline peak to nadir (mg/dL/h) | -113.7 (23.5) | -105.8 (38.8) | -103.0 (28.8) | 8.1 (7.6) | -8.09, 24.24 | 0.304 | 10.59 (7.58) | -5.57, 26.76 | 0.183 |
| AUC(o-iso) (h•mg/dL) | 286.9 (70.1) | 320.0 (89.5) | 338.1 (80.7) | 33.2 (10.9) | 10.10, 56.36 | **0.008** | 50.09 (10.85) | 26.96, 73.23 | **0.000** |
| AUC_{(peak:nadir)} (h•mg/dL) | 161.82 (56.45) | 192.28 (91.94) | 204.58 (86.25) | 30.38 (12.34) | 4.08, 56.67 | **0.026** | 41.57 (12.34) | 15.27, 67.86 | **0.004** |
| Required glycemic rescue (n, %) | 6 (35.3) | 4 (23.5) | 2 (11.8) | - | - | 0.708 a | - | - | 0.225 a |

| **Insulin** | | | | | | | | | |
|---|---|---|---|---|---|---|---|---|---|
| Fasting (µIU/mL) | 4.5 (2.40) | 4.5 (2.7) | 4.1 (2.6) | 0.03 (0.35) | -0.71, 0.78 | 0.922 | -0.43 (0.35) | -1.18, 0.32 | 0.238 |
| Peak (µIU/mL) | 454.5 (240.1) | 349.5 (156.9) | 357.2 (190.9) | -104.5 (43.2) | 196.69, -12.38 | **0.029** | -96.29 (43.24) | 188.45, -4.14 | **0.042** |

| **GLP-1 (pg/mL)** | | | | | | | | | |
|---|---|---|---|---|---|---|---|---|---|
| Fasting (pg/mL) | 12.5 (6.0) | 12.7 (3.4) | 12.71 (4.6) | 0.31 (1.23) | -2.31, 2.94 | 0.803 | 0.28 (1.23) | -2.35, 2.91 | 0.824 |
| Peak (pg/mL) | 326.5 (149.4) | 413.3 (193.6) | 397.1 (150.2) | 87.1 (27.6) | 28.40, 145.84 | **0.006** | 71.20 (27.55) | 12.48, 129.92 | **0.021** |
| AUC(o-iso) (h•mg/dL) | 277.0 (117.0) | 330.8 (140.5) | 351.1 (127.5) | 53.78 (18.0) | 15.48, 92.07 | **0.009** | 74.74 (17.97) | 36.44, 113.03 | **0.001** |

| **Glucagon (pg/mL)** | | | | | | | | | |
|---|---|---|---|---|---|---|---|---|---|
| Fasting (pg/mL) | 156.3 (0) | 156.3 (0) | 156.8 (2.2) | 0.00 (0.37) | -0.79, 0.79 | 1.000 | 0.56 (0.37) | -0.23, 1.34 | 0.153 |
| Peak (pg/mL) | 178.5 (33.3) | 196.7 (49.8) | 182.9 (35.9) | 18.3 (8.4) | 0.29, 36.20 | **0.047** | 4.53 (8.42) | -13.43, 22.48 | 0.599 |
| AUC(₀₋₁₈₀) (h•mg/dL) | 423.5 (108.2) | 466.7 (131.2) | 484.1 (96.7) | 43.6 (27.5) | -14.88, 102.15 | 0.133 | 61.21 (27.45) | 2.69, 119.72 | **0.042** |
| **MEHSS Score** | 14.8 (12.55) | 14.6 (11.90) | 16.5 (14.12) | -0.08 (3.31) | -7.13, 6.97 | 0.98 | 1.69 (3.31) | -5.36, 8.74 | 0.617 |

| | | | | | | | | | |
|---|---|---|---|---|---|---|---|---|---|
| Abbreviations: AUC₀₋₁₈₀, area under the concentration-time curve from 0 to 180 minutes; avexitide 30 q12h, avexitide 30 mg dose every 12 h; avexitide 60 qAM, avexitide 60 mg dose once each morning; Cl, confidence interval; SD, standard deviation; SE, standard error; MEHSS, Modified Edinburgh Hypoglycemia Symptom Scale. Note: mean values are for all data for a given treatment combined. a Two-sided P-value versus placebo (Fisher's exact test). | | | | | | | | | |

The mean rates of hypoglycemia (defined as hypoglycemic symptoms confirmed by an SBGM glucose level <70 mg/dL) were reduced by over 30% and 60% for avexitide 30 mg q12h and avexitide 60 mg qAM, respectively compared to placebo. The LS mean placebo-corrected rate of hypoglycemia was substantially lower during avexitide 30 mg q12h,-1.24 (P= 0.0720) treatment, and significantly lower during avexitide 60 mg qAM, -2.51 (P= 0.0014) treatment (Table 8).

The mean rates of clinically important hypoglycemia (defined as any neuroglycopenic symptoms/signs confirmed by SBGM glucose level <55 mg/dL or as a hypoglycemic episode that required assistance of another person to administer rescue therapy) were reduced by 40% and 59% for avexitide 30 mg q12h and avexitide 60 mg qAM, respectively, compared to placebo. The LS mean placebo-corrected rate of severe hypoglycemia was statistically significantly lower during both dosing regimens of avexitide: avexitide 30 mg q12h,-0.95 (P= 0.0194); avexitide 60 mg qAM, -1.42 (P= 0.0015) (Table 8).

As shown in Table 8, the rates of rescue with oral or G-tube intake were substantially reduced with avexitide. By self-rescue, the LS mean placebo-corrected rescue rates were substantially lower during avexitide 30 mg q12h,-1.60 (P= 0.0614) treatment, and statistically significantly lower during avexitide 60 mg qAM, -3.13 (P= 0.0013) treatment. By third-party assistance, the LS mean placebo-corrected rescue rates were substantially lower with avexitide 30 mg q12h,-0.34 (P= 0.1194) and avexitide 60 mg qAM, -0.38 (P= 0.0856).

While all patients were provided with access to a glucagon emergency kit, no patients used glucagon as a means of hypoglycemia prevention/rescue at any time during the study.

As shown in Figure 6, the mean percent time spent in hypoglycemia during daytime hours (8am-midnight) was substantially reduced during avexitide 30 mg q12h and 60 mg qAM treatments as compared to during placebo treatment. Specifically, the mean percent time with glucose values <70, <55, and <40 mg/dL was reduced by 36%, 49%, and 57%, respectively, during avexitide 30 mg q12h treatment, and by 26%, 24%, and 4%, respectively, during avexitide 60 mg qAM treatment. While the mean percent time spent with glucose levels below CGM thresholds between midnight and 8AM was not substantially different during avexitide treatment versus placebo treatment, a small increase in percent time with glucose levels <55 mg/dL was observed during avexitide 60 mg qAM treatment from 12AM to 4AM, suggesting breakthrough episodes of postprandial hypoglycemia in the early morning hours when avexitide plasma concentrations were waning.

The mean percent time spent with glucose levels >250 mg/dL was less than 1% and similar for all three treatment groups.

### Number of hypoglycemic episodes

The mean number of hypoglycemic episodes sustained for at least 10 minutes during daytime hours (8am-midnight) was substantially reduced during avexitide 30 mg q12h and 60 mg qAM treatments as compared to during placebo treatment. Specifically, the mean number of hypoglycemic episodes (CGM values sustained for at least 10 minutes within a 3-hour period) <70, <55, and <40 mg/dL was reduced by 28%, 43%, and 53% respectively during avexitide 30 mg q12h treatment, and by 23%, 29%, and 24% respectively during avexitide 60 mg qAM treatment. Between midnight and 8AM, the mean number of episodes were not substantially different for avexitide 30 mg q12h and 60 mg qAM treatments versus placebo treatment (Figure 6).

### Quality of Life

Results for the NOS questionnaire indicate that patients treated with avexitide 30 mg q12h and 60 mg qAM compared to placebo, respectively, had fewer instances of loss of consciousness, required less third-party assistance, and had fewer interruptions in their daily activities.

**Table 8. Rate of Hypoglycemia, Clinically Important Hypoglycemia, and Hypoglycemia Rescue**

| **Parameter** | **Mean Value (SD)** | | | **Avexitide 30 q12h (N =17)** | | | **Avexitide 60 qAM (N =17)** | | |
|---|---|---|---|---|---|---|---|---|---|
| | **Placebo (N= 17)** | **Avexitid e 30 q12h (N=17)** | **Avexitid e 60 qAM (N =17)** | **LS Mean Placebo-Corrected Value (SE)** | **95% CI** | **P-value** | **LS Mean Placebo-Corrected Value (SE)** | **95% CI** | **P-value** |
| Rate of hypoglycemia ^{a,c} | 4.03 (3.10) | 2.81 (2.13) | 1.56 (1.27) | -1.24 (0.64) | -2.62, 0.13 | 0.072 | -2.51 (0.643) | -3.878, -1.137 | **0.001** |
| Rate of clinically important hypoglycemia ^{b,c} | 2.42 (2.05) | 1.45 (1.90) | 0.99 (1.11) | -0.95 (0.36) | -1.73, -0.18 | **0.019** | -1.42 (0.364) | -2.192, -0.639 | **0.002** |
| Rate of rescue | 4.87 (3.94) | 3.34 (2.33) | 1.83 (1.62) | -1.6 (0.79) | -3.29, 0.09 | 0.061 | -3.13 (0.793) | -4.818, -1.437 | **0.001** |
| Third-party assistance | 0.63 (1.14) | 0.28 (1.14) | 0.24 (0.75) | -0.34 (0.21) | -0.78, 0.10 | 0.119 | -0.38 (0.206) | -0.818, 0.06 | 0.086 |

| | | | | | | | | | |
|---|---|---|---|---|---|---|---|---|---|
| Abbreviations: avexitide 30 q12h,avexitide 30 mg dose every 12 h; avexitide 60 qAM, avexitide 60 mg dose once each morning; Cl, confidence interval; SD, standard deviation; SE, standard error ^{a} Hypoglycemia was defined as hypoglycemic symptoms confirmed by an SBGM glucose level <70 mg/d. ^{b} Clinically important hypoglycemia was defined as any neuroglycopenic symptoms/signs confirmed by SBGM glucose level <55 mg/dL or as a hypoglycemic episode that required assistance of another person to administer rescue therapy. ^{c} Rates of hypoglycemia and clinically important hypoglycemia were calculated as number of distinct episodes divided by number of days for a given treatment period, then normalized to duration of 2 weeks if the treatment period was not exactly 14 days. Any hypoglycemia events occurring within the same consecutive 3-hour period were counted only as a single hypoglycemia episode, with the lowest glucose value in that 3-hour period being used. ^{d} Rescue is defined as requiring self- or third-party administration of oral or g-tube intake to prevent or treat hypoglycemia. | | | | | | | | | |

Data captured by the NOS questionnaire (designed to evaluate the effect that avexitide treatment has on the occurrence of severe neuroglycopenic outcomes by patient recall at the end of each treatment period) aligned well with eDiary data, revealing fewer instances of loss of consciousness, less requirement for third-party assistance, and fewer interruptions in daily activities while patients were on avexitide treatment as compared to placebo treatment.

**Table 9. Neuroglycopenia Outcome Scale**

| **Parameter ^{a}** | **Number (%) of Patients** | | |
|---|---|---|---|
| | **Treatment** | | |
| | **Placebo (N = 17)** | **Avexitide 30 mg q12h (N = 17)** | **Avexitide 60 mg qAM (N = 17)** |
| Had a Seizure | | | |
| Never | 17 (100.0) | 17 (100.0) | 17 (100.0) |
| Lost Consciousness | | | |
| Never | 15 (88.2) | 17 (100.0) | 17 (100.0) |
| 1 Time | 1 (5.9) | 0 | 0 |
| 2 Times | 1 (5.9) | 0 | 0 |
| Required Help from Third Party | | | |
| Never | 12 (70.6) | 14 (82.4) | 13 (76.5) |
| 1 Time | 1 (5.9) | 1 (5.9) | 1 (5.9) |
| 2 Times | 0 (0.0) | 1 (5.9) | 1 (5.9) |
| 3 Times | 1 (5.9) | 0 | 1 (5.9) |
| 4 or More Times | 3 (17.6) | 1 (5.9) | 1 (5.9) |
| Required Medical Assistance | | | |
| Never | 17 (100.0) | 17 (100.0) | 17 (100.0) |
| Interrupted Daily Activities | | | |
| Never | 2 (11.8) | 3 (17.6) | 5 (29.4) |
| 1 Time | 1 (5.9) | 3 (17.6) | 0 |
| 2 Times | 1 (5.9) | 1 (5.9) | 3 (17.6) |
| 3 Times | 2 (11.8) | 3 (17.6) | 3 (17.6) |
| 4 or More Times | 11 (64.7) | 7 (41.2) | 6 (35.3) |

| | | | |
|---|---|---|---|
| ^{a} Describes subject's experience over the past two weeks. | | | |

### EXAMPLE 5

### Event Adjudication

In certain aspects of a study with an endpoint measuring severe hypoglycemia events, particularly in cases where external assistance was needed but not received, the following delineates procedures for the adjudication of severe hypoglycemia events.

### Severe Hypoglycemia Event Definition/Criteria

The American Diabetes Association (ADA) "Standards of Medical Care in Diabetes" recommends the classification of hypoglycemia shown in Table 10 (ADA 2019; Agiostratidou et al 2017).

**Table 10. ADA Hypoglycemia Classification**

| | |
|---|---|
| Level 1 | A measurable glucose concentration <70 mg/dL (3.9 mmol/L) but ≥54 mg/dL (3.0 mmol/L). A blood glucose concentration of 70 mg/dL (3.9 mmol/L) has been recognized as a threshold for neuroendocrine responses to falling glucose in people without diabetes. |
| Level 2 | A blood glucose concentration <54 mg/dL [3.0 mmol/L]). This is the threshold at which neuroglycopenic symptoms begin to occur and requires immediate action to resolve the hypoglycemic event. |
| Level 3 | A severe event characterized by altered mental and/or physical functioning that requires assistance from another person for recovery. |

The trial will capture Level 2 and Level 3 hypoglycemia as well as signs and symptoms of hypoglycemia-induced central nervous system (CNS) impairment (neuroglycopenia signs/symptoms specific to cognitive/physical dysfunction) primarily via patient electronic diaries.

Level 3, severe hypoglycemia, is a subset of hypoglycemia-induced CNS impairment and indicates a high level of dysfunction. It is important to note that although patients may require assistance, they may not always receive it (for example assistance may not be available).

Since the categorization of a severe hypoglycemia event is somewhat subjective particularly in cases where external assistance was needed but not received, all potential severe hypoglycemia events will be adjudicated per the definition and the criteria outlined below.

Criteria for determining whether 3^{rd} party assistance was required:
- Patient is unable to independently obtain rescue therapy and requires help from another person. This includes preparing, retrieving or administering rescue therapy.
- Patient experiences any of the following neuroglycopenic outcomes: seizure (focal or generalized); loss of consciousness (LOC); obtundation/coma; stroke-like signs or symptoms including motor deficit and/or speech deficit, etc.
- Patient experiences a neuroglycopenic outcome and requires external assistance to prevent injury.
- Patient requires hypoglycemia rescue by medical professionals (emergency medical personnel, etc).
- Patient experiences an adverse event (AE) in which upon review of source documents, hypoglycemia appears to have contributed. The following AEs will be investigated: accidents, injuries, falls, loss of consciousness/syncope, seizures, hypoglycemia.
- Patient is hospitalized for hypoglycemia.
- Patient has a measured BG concentration <40 mg/dL.

### EXAMPLE 6

The study outlined in Example 2, a total of 18 patients were enrolled: 8 were randomly assigned to the placebo/avexitide 30 mg q12h/avexitide 60 mg qAM sequence and 10 to the placebo/avexitide 60 mg qAM/avexitide 30 mg q12h sequence. All 18 completed each treatment period and the study.

All patients were female. Most were white (94.4%) and not Hispanic or Latino (88.9%). Mean weight, height, and BMI were 81 kg, 165 cm, and 30 kg/m2, respectively.

The overall mean time since RYGB surgery was 93.5 months (range 16 - 198), and the overall mean time from RYGB surgery to first experience of hypoglycemic symptoms was 35.7 months (range 2 - 144 months). Almost half of the patients had experienced episodes of hypoglycemia-induced loss of consciousness (44.4%). Nearly all patients (94.5%) experienced either daily (7, 38.9%) or weekly (10, 55.6%) hypoglycemic symptoms; 1 patient (5.6%) reported monthly symptoms. Three patients (16.7%) had been hospitalized due to hypoglycemia; 2 patients (11.1%) reported a history of hypoglycemia-induced seizure of which 1 patient (5.6%) reported monthly seizures.

Most patients had been following medical nutritional therapy (currently accepted guideline for treatment of PBH) for years. During the Run-In Period, all eligible patients were confirmed to be refractory to medical nutrition therapy; per protocol, all patients continued dietary treatment during study. Most patients (15, 83.3%) had received pharmacological therapy with the off-label use of at least one medication for their PBH, and 3 patients had undergone additional surgical procedures (placement of gastrostomy tube in 2 patients; RYGB revision in 1 patient) to treat their PBH. None of the patients had a history of Type 2 diabetes.

### Extent of Exposure and Treatment Adherence

Assigned treatments were administered during almost all the days of the placebo, avexitide 30 mg q12h, and avexitide 60 mg qAM treatment periods (mean number of days, 14.3, 13.8, and 14.3, respectively). Adherence to the study treatment regimens was high: mean, 96.3%, 99.9%, and 98.3%, respectively.

### Analysis of Endpoints

Magnitude of Postprandial Hypoglycemia: The primary efficacy endpoint was met by both avexitide regimens. The LS mean placebo-corrected postprandial plasma glucose nadir during MMTT provocation showed statistically significant increases in glucose nadir for both avexitide 30 mg q12h (LS mean = 10.10 mg/dL, p = 0.0011) and avexitide 60 mg qAM (LS mean = 12.19 mg/dL, p = 0.0002).

Peak Postprandial Insulin Response: The LS mean placebo-corrected postprandial insulin peak during MMTT provocation showed statistically significant decreases in insulin peak with avexitide 30 mg q12h (LS mean = -104.53 µIU/mL, p = 0.0288) and avexitide 60 mg qAM (LS mean = -96.29 µIU/mL, p = 0.0417).

Requirement for Glycemic Rescue: A smaller proportion of patients required glycemic rescue in response to MMTT provocation during avexitide 30 mg q12h (23.5%) and 60 mg qAM (11.8%) treatments than during placebo (35.3%) treatment.

Postprandial Neuroglycopenic Signs and Symptoms: There were no statistically significant differences in the reported experience of postprandial neuroglycopenic symptoms during MMTT provocation as measured by the MEHSS.

Rate of Hypoglycemia: The LS mean placebo-corrected rate of hypoglycemia was substantially lower during avexitide 30 mg q12h treatment, -1.24 (p = 0.0720) and significantly lower during avexitide 60 mg qAM treatment, -2.51 (p = 0.0014).

Rate of Severe Hypoglycemia: The LS mean placebo-corrected rate of severe hypoglycemia was statistically significantly lower during both dosing regimens of avexitide: avexitide 30 mg q12h, -0.95 (p = 0.0194); avexitide 60 mg qAM, -1.42 (p = 0.0015).

Rate of Hypoglycemia Prevention/Rescue with Glucagon: No patients used glucagon as a means of hypoglycemia prevention or rescue at any time during the study.

Rate of Hypoglycemia Prevention/Rescue with Oral/G-Tube Intake: The LS mean placebo-corrected self-rescue rates were substantially reduced with avexitide 30 mg q12h, -1.60 (p = 0.0614), and significantly reduced with avexitide 60 mg qAM, -3.13 (p = 0.0013). The LS mean placebo-corrected third-party assistance rescue rates were substantially reduced with avexitide 30 mg q12h, -0.34 (p = 0.1194) and avexitide 60 mg qAM, -0.38 (p = 0.0856).

Percent of Time with Glucose Measures <70 mg/dL, <55 mg/dL, or <40 mg/dL between 8AM and midnight and between midnight and 8AM as measured by CGM:
- Between 8AM and midnight, the mean percent time spent with glucose levels below CGM thresholds was lower during avexitide 30 mg q12h and avexitide 60 mg qAM treatments versus placebo treatment, respectively: <70 mg/dL, 5.0% and 5.8% versus 7.8%; <55 mg/dL, 1.1% and 1.6% versus 2.1%; <40 mg/dL, 0.23% and 0.52% versus 0.54%.
- Between midnight and 8AM, the mean percent time spent with glucose levels below CGM thresholds was not substantially different in the avexitide 30 mg q12h and 60 mg qAM treatments versus placebo treatment, respectively: <70 mg/dL, 9.0% and 11.9% versus 9.7%; <55 mg/dL, 2.2% and 3.2% versus 2.4%; and <40 mg/dL, 0.53% and 0.82% versus 0.54%.

Percent of Time with Glucose Measures >180 mg/dL and ≤250 mg/dL, or >250 mg/dL as measured by CGM: The mean percent time with glucose levels >180 mg/dL and ≤250 mg/dL was higher for avexitide 30 mg q12h (4.0%) and avexitide 60 mg qAM (4.3%) versus placebo (2.8%). Percent time with glucose levels >250 mg/dL was less than 1% and similar during avexitide and placebo treatment.

Fasting Glucose, Peak Glucose, Peak-to-Nadir Difference in Glucose, the Rate of Glucose Decline from Peak to Nadir, and the AUC Glucose During MMTT Provocation:
- Mean fasting glucose remained normal during avexitide 30 mg q12h ID (87.5 mg/dL), avexitide 60 mg qAM (84.6 mg/dL), and placebo (83.8 mg/dL) treatment.
- The mean plasma glucose peak was similar during avexitide 30 mg q12h (218.2 mg/dL) treatment and higher during avexitide 60 mg qAM (224.5 mg/dL) treatment compared to placebo (211.8 mg/dL).
- The time to glucose peak was similar during avexitide 30 mg q12h (37.9 min) treatment and shorter during avexitide 60 mg qAM (34.4 min) treatment compared to placebo (39.9 min) treatment.
- The mean peak-to-nadir differences in glucose levels were similar:
   ▪ avexitide 30 mg q12h (-161.1 mg/dL); avexitide 60 mg qAM (-165.4 mg/dL); and
   ▪ placebo (-164.6 mg/dL).
- The AUC glucose between 0 to 3 hours was higher during avexitide 30 mg q12h (319.96 h•mg/dL) and 60 mg qAM (338.06 h•mg/dL) treatments compared to placebo (286.89 h•mg/dL) treatment.

Fasting and Postprandial GLP-1 and Glucagon Levels During the MMTT:
- Fasting GLP-1 levels were similar across the avexitide 30 mg q12h and 60 mg qAM treatments versus placebo treatment (12.7 and 12.7 versus 12.5 pg/mL, respectively). Peak GLP-1 levels were higher in the avexitide groups (413 and 397 versus 326 pg/mL), as were the GLP-1 AUC values during the MMTT (0 to 180 minutes, 331 and 351 versus 277 h•pg/mL).
- Fasting glucagon levels were similar across the avexitide 30 mg q12h and 60 mg qAM treatments versus placebo treatment (156 and 157 versus 156 pg/mL, respectively). Peak glucagon levels were higher in the avexitide groups (197 and 183 versus 179 pg/mL), as were the glucagon AUC values during the MMTT (0 to 180 minutes, 467 and 484 versus 423 h•pg/mL).

QOL as Measured by Questionnaires:
- Across the NSS, HIS, HSIS, and SF-36v2 questionnaires, improvements were observed during placebo and avexitide treatment periods with no clear differences observed.
- Results for the NOS questionnaire indicate that patients treated with avexitide 30 mg q12h and 60 mg qAM compared to placebo, respectively, had fewer instances of loss of consciousness, required less third-party assistance, and had fewer interruptions in their daily activities.

KPS values were similar during the avexitide 30 mg q12h and 60 mg qAM treatments compared to placebo treatment (83.5 and 87.1 versus 82.4, respectively).

### Pharmacokinetics (PK)

Evidence of systemic plasma exposure to avexitide was observed in all patients following repeat SC doses of avexitide 30 mg q12h and avexitide 60 mg qAM.

Mean predose plasma avexitide levels were higher during avexitide 30 mg q12h treatment compared to during avexitide 60 mg qAM treatment.

No clear difference was observed in mean Tₘₐₓ values of avexitide following SC administration of avexitide 30 mg q12h (5.06 h) and avexitide 60 mg qAM (5.88 h).

Plasma exposure (C_{avg}, Cₘₐₓ, and AUC₀₋ₜ) following avexitide 60 mg qAM treatment was consistently greater than that observed following avexitide 30 mg q12h treatment: Mean plasma Cₘₐₓ values following avexitide 30 mg q12h and avexitide 60 mg qAM treatment were 357 versus 613 ng/mL, respectively; mean plasma AUC₀₋ₜ values following avexitide 30 mg q12h and avexitide 60 mg qAM treatment were 2035 versus 2854 ng•h/mL, respectively. A similar trend was noted for mean plasma C_{avg} values.

### Safety

All TEAEs - A similar proportion of patients experienced TEAEs during placebo treatment (77.8%) and during avexitide 60 mg qAM treatment (72.2%), whereas a smaller proportion experienced TEAEs during avexitide 30 mg q12h treatment (38.9%). The most common AEs (reported in ≥20% of patients) in the placebo, avexitide 30 mg q12h, and avexitide 60 mg qAM regimens, respectively, were injection site bruising (38.9%, 0, and 5.6%), headache (22.2%, 5.6%, and 5.6%), and nausea (22.2%, 11.1%, and 16.7%).

Intensity of TEAEs - The maximal TEAE intensity was mild for 33.3% of patients, moderate for 44.4%, and severe for 11.1%. A total of 68 TEAEs were reported, and most events were mild (43, 63%) or moderate (23, 34%), with 2 severe events (2, 3%), each of which were considered unrelated to procedure or study drug, and each resolved with treatment.

Deaths, Serious, and Significant TEAEs:
- No deaths or treatment-related serious TEAEs occurred.
- No patients discontinued study as a result of a TEAE.
- One SAE of severe presyncope considered unrelated to study treatment or procedure was reported.

Clinical Laboratory Tests and Physical Findings - No clinically significant changes were noted in chemistry or hematology laboratory results, vital signs, or body weight changes.

Antidrug Antibodies - One patient developed low positive titers for antidrug antibodies; no TEAEs were associated with this finding.

Avexitide 30 mg q12 h for 14 days and 60 mg qAM for 14 days as compared to placebo administered for 14 days demonstrated consistent improvements across multiple clinical and metabolic parameters as measured in the inpatient setting with MMTT provocation and in the outpatient setting with eDiary, SBGM, and blinded CGM. Most notably, avexitide treatment as compared to placebo resulted in the following clinical and metabolic findings:

Increase in postprandial glucose nadir during MMTT provocation.

Reduction in postprandial peak insulin during MMTT provocation.

Reduction in the requirement for rescue during MMTT provocation.

Reduction in the rates of hypoglycemia and severe hypoglycemia as collected by eDiary.

Reduction in the rates of hypoglycemia rescue as collected by eDiary.

Reduction in the percent diurnal time in hypoglycemia as documented by CGM.

Reduction in the number of diurnal hypoglycemic episodes as documented by CGM.

While the PK profile could not be fully characterized, avexitide 30 mg q12h appeared to confer more sustained plasma avexitide exposure with potentially improved late evening and early morning (predose) coverage, while avexitide 60 mg qAM provided higher daytime exposure.

Both dosing regimens were well-tolerated, with no treatment-related serious adverse events, and no participant withdrawals. Adverse events were typically mild and transient, and no clinically significant safety signals were observed.

Overall, the strength and consistency of the data in aggregate combined with the tolerability and safety data demonstrate a clear and robust treatment benefit and support the continued development of avexitide for treatment of PBH.

### EXAMPLE 7

Avexitide 90 mg/mL is a sterile, clear, colorless solution and is supplied in single-use 3 mL vials containing 0.5 mL solution. The drug product composition consists of 90 mg/mL avexitide (active ingredient) in a sodium acetate buffer containing mannitol as an excipient. A total of 0.5 mL solution can be withdrawn from the vial for SC injection.

Avexitide injection is administered subcutaneously in the anterolateral aspect of the thigh.

An endpoint will be a reduction in the rate of hypoglycemia-induced central nervous system (CNS) impairment for avexitide in post-bariatric hypoglycemia (PBH). Unlike other tissues (muscle, liver, etc.), the CNS requires a continuous supply of glucose. The brain can increase its rate of glucose transport, but this compensatory mechanism may be inadequate to maintain adequate glucose concentrations in the brain if the blood glucose level is extremely low.

When the CNS is deprived of glucose the following signs and symptoms may occur:
- Cognitive deterioration, abnormal mentation, impaired judgement
- Personality/behavior change, bizarre behavior, emotional lability
- Confusion, amnesia, delirium
- Vision change, blurred vision, double vision
- Difficulty speaking, slurred speech, nonsensical speech
- Coordination difficulties, ataxia
- Focal or general motor deficit
- Excessive sleepiness (somnolence), stupor, presyncope
- Loss of consciousness
- Convulsions, generalized or focal seizures; and/or
- Coma

In the study outlined in Example 2, when a patient reported hypoglycemic symptoms, a corresponding blood glucose confirmed hypoglycemia. A total of 364 symptomatic episodes were recorded of which 81% were associated with a finger stick blood glucose < 70 mg/dL and 38% were associated with a BG < 54mg/dL. The signs and symptoms most closely associated with the lowest blood glucose concentrations included loss of consciousness (LOC), slurred speech, visual changes (blurred vision, double vision), bizarre behavior, confusion and coordination difficulties.

All events of LOC and slurred speech were associated with very low BG concentrations (LOC: 31, 42, 45 and 52 mg/dL; slurred speech: 45 mg/dL). Blood glucose concentrations < 54 mg/dL were confirmed in 66% of events when visual changes were reported, 64% when bizarre behavior was reported, 58% when confusion was reported and 56% when coordination difficulties were reported. Conversely these signs/symptoms were rarely reported when BG concentration was > 70 mg/dL (4 - 9%). The average BG concentration was 54mg/dL for events in which any of the following signs/symptoms were reported: slurred speech; LOC; visual changes; bizarre behavior; confusion or coordination difficulties. When none of these were present, the average BG was 64 mg/dL. Using these terms, we were able to identify 83 (61%) out of 137 reported hypoglycemic events with confirmed BG < 54 mg/dL in the Example 2 study. A summary of these results in provided in Table 11.

**Table 11.**

| BG (mg/dL) | Visual Changes | Bizarre Behavior | Confusion | Coordination Difficulties | Received Assistance | Drowsy | Lightheaded/Dizzy |
|---|---|---|---|---|---|---|---|
| <54 (137) events) | 47 | 7 | 62 | 23 | 31 | 38 | 87 |
| ≥54 and <70 (157 events) | 21 | 3 | 37 | 16 | 13 | 49 | 65 |
| ≥70 (70 events) | 3 | 1 | 9 | 2 | 1 | 19 | 11 |
| Total episodes = 364 | =71 | =11 | =108 | =41 | =45 | =106 | =163 |

Efficacy endpoint of the reduction of hypoglycemia-induced CNS impairment where any of the following signs/symptoms denotes CNS impairment:
- Cognitive deterioration, abnormal mentation, impaired judgement
- Personality/behavior change, bizarre behavior, emotional lability
- Confusion, amnesia, delirium
- Vision change, blurred vision, double vision
- Difficulty speaking, slurred speech, nonsensical speech
- Coordination difficulties, ataxia
- Focal or general motor deficit
- Excessive sleepiness (somnolence), stupor, presyncope
- Loss of consciousness
- Convulsions, generalized or focal seizures; and/or
- Coma

Reduction in the number of events of severe hypoglycemia is also an endpoint, and all potential severe hypoglycemia events will be adjudicated.

Although the foregoing invention has been described in some detail by way of illustration and example for purposes of clarity of understanding, one of skill in the art will appreciate that many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with the true scope and spirit of the invention being indicated by the following claims.

All publications, patents, patent applications or other documents cited herein are hereby incorporated by reference in their entirety for all purposes to the same extent as if each individual publication, patent, patent application, or other document was individually indicated to be incorporated by reference for all purposes.

### RELEVANT PARAGRAPHS:

1. A method of treating one or more of hyperinsulinemic hypoglycemia (HH), fasting hyperinsulinemic hypoglycemia, insulinoma, or hypoglycemia/NIPHS, the method comprising subcutaneously administering to the subject a buffered liquid formulation comprising avexitide at a concentration of between 2 - 90 mg/mL.
2. The method of paragraph 1, wherein the avexitide is administered less than 30 days.
3. The method of paragraph 1 or 2, wherein the avexitide is administered until one or both of glucose level and insulin level are stabilized.
4. The method of any one of paragraphs 1-3, wherein HH comprises post-bariatric hypoglycemia (PBH).
5. The method of any one of paragraphs 1-4, wherein the subject has previously had one or more of Roux-en-Y gastric bypass surgery, vertical sleeve gastrectomy (VSG), biliopancreatic diversion (BPD), upper-GI procedure, gastrectomy, esophagectomy, Nissen fundoplication, gastric bypass surgery, vagotomy with pyloroplasty, vagotomy without pyloroplasty, or bilroth.
6. The method of any one of paragraphs 1-3, wherein the subject has congenital microgastria or a condition that speeds transit to the ileum.
7. The method of paragraph 1, wherein the avexitide is administered at a total daily dose from about 40 mg to about 90 mg.
8. The method of paragraph 1, wherein the avexitide is administered at a total daily dose from about 40 mg to about 120 mg; or from 30 mg - 60 mg BID, or from 60 mg - 120 mg QD; or from about 45 mg BID, or about 35 - 55 mg BID.
9. The method of paragraph 1, wherein the avexitide is administered at a total daily dose is about 90 mg; or about 90 mg QD, or about 60 to 120 mg/day.
10. The method of paragraph 1, wherein the subject is administered 0.375 mg/kg/dose or 0.56 mg/kg/dose or 0.75 mg/kg/day or about 1.125 mg/kg/day, or about 0.3 - 1.6 mg/kg/dose of avexitide BID; or about 0.6-3.2 mg/kg/day BID or about 0.2 -1.2 mg/kg/dose, or about 0.6-3.6 mg/kg/day BID; or about 0.6-3.6 mg/kg/day TID.
11. The method of paragraph 1, wherein the avexitide is administered by IV infusion at about 0.1 to 1.0 mg/kg/hr, or at about 0.2 to 0.6 mg/kg/hr.
12. The method of paragraph 2, wherein the avexitide is administered at a total daily dose of about 60 or about 120 mg.
13. The method of paragraph 3, wherein the avexitide is administered once daily (QD) or twice daily (BID).
14. The method of paragraph 1, wherein the subject is administered avexitide at about 30 mg to about 90 mg BID, and wherein the subject is not required to fast after dosing or is not required to delay a first meal of the day.
15. The method of paragraph 1, wherein the avexitide is administered at 45 mg - 120 mg QD, and wherein the subject is required to fast after dosing or to delay the first meal of the day.
16. The method of paragraph 15, wherein the delay is from between about 30 minutes to about 1.5 hours.
17. The method of paragraph 15, wherein the subject is required to fast overnight before a morning dose.
18. The method of paragraph 15, wherein the subject is required to fast for about 45 - 90 minutes after a morning dose.
19. The method of paragraph 15, wherein the subject is required to fast overnight before a morning dose and for approximately 45 - 90 minutes after the morning dose.
20. The method of paragraph 1, wherein the avexitide is administered at a dose of 30 mg BID or 45 mg BID.
21. The method of paragraph 20, wherein the method comprises administering a morning dose and an evening dose, and wherein the evening dose is administered about 12 hours after the morning dose.
22. The method of paragraph 21, wherein the morning dose is administered at least about 30 minutes to about 60 minutes before a meal.
23. The method of paragraph 1, wherein the avexitide is administered at a dose of 60 mg QD.
24. The method of paragraph 23, wherein the avexitide is administered in the morning or in proximity to a first meal of the day.
25. The method of paragraph 24, wherein the avexitide is administered at least about 30 minutes to about 90 minutes before a first meal of a day.
26. The method of any of paragraphs 1-25, wherein the formulation comprises avexitide at a concentration from 30 mg/mL to 180 mg/mL; or from 2 mg/mL to 29 mg/mL.
27. The method of any of paragraphs 1 to 25, wherein the formulation comprises avexitide at a concentration from between 45 mg/ml and 90 mg/ml.
28. The method of paragraph 26, wherein the formulation comprises avexitide at a concentration of 30 mg/mL.
29. The method of paragraph 26, wherein the formulation comprises avexitide at a concentration of 90 mg/mL.
30. The method of any of paragraphs 1 to 29, wherein the formulation has a pH of about 5.5.
31. The method of any of paragraphs 1 to 30, wherein the formulation comprises a sodium acetate buffer.
32. The method of any of paragraphs 1 to 31, wherein the formulation comprises a tonicity modifier.
33. The method of paragraph 32, wherein the tonicity modifier comprises mannitol.
34. The method of any of paragraphs 1-33, wherein avexitide is administered for 30 days or less or at least 30 days.
35. The method of any of paragraphs 1-34, wherein treatment reduces the number and/or severity of postprandial neuroglycopenic symptoms in the subject.
36. The method of any of paragraphs 1-35, wherein treatment improves postprandial glucose nadir for the subject.
37. The method of any of paragraphs 1-36, wherein treatment reduces postprandial insulin peak for the subject.
38. The method of any of paragraphs 1-37, wherein treatment reduces the rate of hypoglycemia in the subject.
39. The method of paragraph 1, wherein the administration reduces the number and/or severity of postprandial neuroglycopenic symptoms in the subject.
40. The method of paragraph 1, wherein the administration improves postprandial glucose nadir for the subject.
41. The method of paragraph 40, wherein there is a significant increase the mixed meal tolerance test ("MMTT") glucose nadir for the subject.
42. The method of paragraph 41, wherein the mean plasma glucose nadir during MMTT provocation increase by 21% - 25% for the subject compared to a control.
43. The method of paragraph 40, wherein the subject requires fewer glycemic rescues after treatment as compared to a control.
44. The method of paragraph 39, wherein peak postprandial glucose values in the subject increase, and wherein this corresponds to a significantly accelerated time to glucose peak during avexitide administration.
45. The method of paragraph 1, wherein the administration reduces postprandial insulin peak in the subject.
46. The method of paragraph 45, wherein mean postprandial insulin peak during an MMTT provocation is reduced from about 21% to 23% in the subject compared to a control.
47. The method of paragraph 1, wherein peak GLP-1 and glucagon levels are higher in the subject as compared to a control
48. The method of paragraph 1, wherein GLP-1 and glucagon AUC values are higher during the MMTT in the subject as compared to a control.
49. The method of paragraph 1, wherein the subject experiences less frequent hypoglycemic episodes during avexitide treatment as compared to a control.
50. The method of paragraph 1, wherein the administration reduces the rate of hypoglycemia in the subject.
51. The method of paragraph 1, wherein the administration reduces the rate of severe hypoglycemia in the subject.
52. The method of paragraph 1, wherein the administration reduces severe hypoglycemia in the subject.
53. The method of paragraph 52, wherein severe hypoglycemia comprises symptoms requiring the subject to receive third party assistance.
54. The method of paragraph 1, wherein the administration reduces the rate of hypoglycemia-induced CNS impairment in the subject.
55. The method of paragraph 1, wherein the administration provides clinically meaningful improvement in the subject in one or more of the rate of hypoglycemia induced CNS impairment, severe hypoglycemia, postprandial insulin peak, rate of severe hypoglycemia, or postprandial glucose nadir.
56. The method of paragraph 1, wherein the administration results in an improvement in postprandial hypoglycemia in the subject.
57. The method of paragraph 1, wherein the administration results in an increase in postprandial plasma glucose nadir in the subject as compared to baseline or a control.
58. The method of paragraph 1, wherein the administration results in an improvement in postprandial neuroglycopenic symptoms or hypoglycemic symptoms in the subj ect.
59. The method of paragraph 1, wherein the avexitide is administered twice daily (BID) within about 60 minutes prior to morning and evening meals, or prior to two main meals of the day for the subject.
60. The method of paragraph 1, wherein the avexitide is administered BID and at least about 6 hours apart.
61. The method of paragraph 1, wherein the avexitide is administered in a total volume ranging from 0.25-2.0 ml; or in a total volume ranging from about 0.05-0.1 ml; or in an injection volume ranging from 0.25-1.5 ml, or from 0.5-1 ml, or from 0.7-1 ml, or from 0.05-0.1 ml.
62. The method of any of paragraphs 1-61, wherein treatment reduces the rate of severe hypoglycemia in the subject.
63. The method of any of paragraphs 1-62, wherein treatment reduces the rate of hypoglycemia-induced CNS impairment in the subject.
64. The method of paragraph 63, wherein hypoglycemia-induced CNS impairment includes one of more of the following signs or symptoms: cognitive deterioration, abnormal mentation, impaired judgement, personality/behavior change, bizarre behavior, emotional lability, confusion, amnesia, delirium, vision change, blurred vision, double vision, difficulty speaking, slurred speech, nonsensical speech, coordination difficulties, ataxia, focal or general motor deficit, excessive sleepiness (somnolence), stupor, presyncope, loss of consciousness, convulsions, generalized or focal seizures, or coma.
65. The method of paragraph 63, wherein the signs and symptoms associated with lowest blood glucose concentrations comprise one or more of loss of consciousness (LOC), slurred speech, visual changes (blurred vision, double vision), bizarre behavior, confusion, and coordination difficulties.
66. The method of paragraph 1, wherein the subject is refractory to dietary treatment.
67. The method of paragraph 1, wherein the avexitide is administered at a dose of 45 mg BID.
68. The method of any of paragraphs 17-19 and 21, wherein the morning dose is administered from between about 30 minutes to about 90 minutes before a meal.
69. The method of any of paragraphs 1-68, wherein treatment reduces the rate of rescue needed by the subject.
70. The method of paragraph 69, wherein rescue comprises administering glucagon to the subject at the earlier of glucose ≤ 50 mg/dL + neuroglycopenia or glucose ≤ 40 mg/dL +/- neuroglycopenia.
71. The method of paragraph 69, wherein the subject is experiencing daily or weekly symptoms of hypoglycemia.
72. The method of paragraph 1, wherein the percent time in Hypoglycemia by CGM threshold for the subject is reduced after administration.
73. The method of paragraph 1, wherein the number of episodes of hypoglycemia by CGM threshold for the subject is reduced after administration.
74. The method of paragraph 1, wherein there were fewer interruptions in daily activities for the subject as compared to a control.
75. The method of paragraph 1, wherein the mean rates of hypoglycemia in the subject are reduced from between about 30% - 60% for avexitide as compared to a control.
76. The method of paragraph 75, wherein rates of hypoglycemia are an SBGM glucose level <70 mg/dL.
77. The method of paragraph 1, wherein the mean rates of clinically important hypoglycemia in the subject are reduced by between about 40% - 59% as compared to a control.
78. The method of paragraph 77, wherein the clinically important hypoglycemia are any neuroglycopenic symptoms/signs confirmed by SBGM glucose level <55 mg/dL or as a hypoglycemic episode that required assistance of another person to administer rescue therapy.
79. The method of paragraph 1, wherein the rates of rescue with oral or G-tube intake for the subject are substantially reduced.
80. The method of paragraph 1, wherein the mean percent time spent in hypoglycemia during daytime hours (8am-midnight) for the subject is substantially reduced as compared to a control.
81. The method of paragraph 80, wherein the mean percent time with glucose values <70 mg/dL, <55 mg/dL, and <40 mg/dL for the subject is reduced by between about 4 - 57% as compared with a control.
82. The method of paragraph 80, wherein the mean percent time with glucose values <70 mg/dL, <55 mg/dL, and <40 mg/dL for the subject is reduced by between about 36 - 57% as compared with a control.
83. The method of paragraph 80, wherein the mean percent time with glucose values <70 mg/dL, <55 mg/dL, and <40 mg/dL for the subject is reduced by between about 4 - 26% as compared with a control
84. The method of paragraph 1, wherein the mean number of hypoglycemic episodes sustained for at least 10 minutes during daytime hours (8am-midnight) for the subject is substantially reduced as compared to a control.
85. The method of paragraph 85, wherein the mean number of hypoglycemic episodes (CGM values sustained for at least 10 minutes within a 3-hour period) <70 mg/dL, <55 mg/dL, and <40 mg/dL for the subject is reduced by about 28% - 53% or by about 23% - 29% as compared to a control.
86. The method of any of paragraphs 42, 43, 46-49, 57, 74, 75, 77, and 81-86, wherein the control comprises a comparison to the subject's postprandial plasma glucose nadir prior to the onset of treatment or as measured during MMTT.
87. The method of any of paragraphs 42, 43, 46-49, 57, 74, 75, 77, and 81-86, wherein the control comprises a comparison to a control subject that is not administered the buffered liquid formulation.

## Claims

1. A buffered liquid formulation comprising avexitide for use in reducing the rate of hypoglycemia events in a subject with hyperinsulinemic hypoglycemia (HH), wherein the buffered liquid formulation is subcutaneously administered; or
a buffered liquid formulation comprising avexitide for use in substantially reducing, as compared to a control subject that is not administered the formulation, the mean percent time spent in hypoglycemia in a subject with hyperinsulinemic hypoglycemia (HH), wherein the buffered liquid formulation is subcutaneously administered; or
a buffered liquid formulation comprising avexitide for use in substantially reducing, as compared to a control subject that is not administered the formulation, the mean number of hypoglycemic episodes in a subject with hyperinsulinemic hypoglycemia (HH), wherein the buffered liquid formulation is subcutaneously administered.

2. The buffered liquid formulation comprising avexitide for use in substantially reducing, as compared to a control subject that is not administered the formulation, the mean percent time spent in hypoglycemia in a subject with hyperinsulinemic hypoglycemia (HH) according to claim 1, wherein:
i) the mean percent time with glucose values <70 mg/dL, <55 mg/dL, and <40 mg/dL for the subject is reduced by from 4 to 57% as compared with a control that is not administered the formulation; or
ii) the mean percent time with glucose values <70 mg/dL, <55 mg/dL, and <40 mg/dL for the subject is reduced by from 36 to 57% as compared with a control that is not administered the formulation; or
iii) the mean percent time with glucose values <70 mg/dL, <55 mg/dL, and <40 mg/dL for the subject is reduced by from 4 to 26% as compared with a control that is not administered the formulation.

3. The buffered liquid formulation comprising avexitide for use in substantially reducing, as compared to a control subject that is not administered the formulation, the mean number of hypoglycemic episodes in a subject with hyperinsulinemic hypoglycemia (HH) according to claim 1, wherein:
i) a hypoglycemic episode is a blood glucose level sustained for at least 10 minutes below 70 mg/dL, below 55 mg/dL, or below 40 mg/dL; and/or
ii) the mean number of hypoglycemic episodes for the subject is reduced by from 28% to 53% or from 23% to 29% as compared to a control that is not administered the formulation.

4. The buffered liquid formulation for use according to any preceding claim, wherein:
i) the avexitide is administered at a total daily dose from about 40 mg to about 120 mg; and/or
ii) the formulation is administered once daily (QD) or twice daily (BID); and/or
iii) the avexitide is administered at a dose from 30 mg to 60 mg BID; from 60 mg to 120 mg QD; or from 35 mg to 55 mg BID; and/or
iv) the avexitide is administered at a dose of about 90 mg QD or about 45 mg BID.

5. The buffered liquid formulation for use according to any preceding claim, wherein:
i) the formulation comprises avexitide at a concentration from 2 mg/ml to 180 mg/ml; and/or
ii) the formulation comprises avexitide at a concentration from 30 mg/ml to 180 mg/ml;
and/or
iii) the formulation is administered for 30 days or less; and/or
iv) the formulation is administered until one or both of glucose level and insulin level are stabilized; and/or
v) HH comprises post-bariatric hypoglycemia (PBH), fasting HH, insulinoma, or hypoglycemia/NIPHS; and/or
vi) the subject has previously had one or more of Roux-en-Y gastric bypass surgery, vertical sleeve gastrectomy (VSG), biliopancreatic diversion (BPD), upper-GI procedure, gastrectomy, esophagectomy, Nissen fundoplication, gastric bypass surgery, vagotomy with pyloroplasty, vagotomy without pyloroplasty, or bilroth; and/or
vii) the subject has congenital microgastria or a condition that speeds transit to the ileum.

6. The buffered liquid formulation for use according to any preceding claim, wherein the avexitide is administered at 0.375 mg/kg/dose, 0.56 mg/kg/dose, 0.75 mg/kg/day, or 1.125 mg/kg/day, or 0.3-1.6 mg/kg/dose of avexitide BID; or about 0.6-3.2 mg/kg/day BID or about 0.2 - 1.2 mg/kg/dose, or about 0.6-3.6 mg/kg/day BID; or about 0.6-3.6 mg/kg/day TID.

7. The buffered liquid formulation for use according to any of claims 1 to 4 or 6, wherein the subject is administered avexitide at a dose from 30 mg to 90 mg BID, and wherein the subject is not required to fast after dosing or is not required to delay a first meal of the day.

8. The buffered liquid formulation for use according to any of claims 1 to 4 or 6, wherein the avexitide is administered at a dose from 45 mg to 120 mg QD, and wherein the subject is required to fast after dosing or to delay the first meal of the day.

9. The buffered liquid formulation for use according to claim 8, wherein:
i) the delay is 30 minutes to 1.5 hours; or
ii) the subject is required to fast overnight before a morning dose; or
iii) the subject is required to fast for 45 minutes to 90 minutes after a morning dose; or
iv) the subject is required to fast overnight before a morning dose and for 45 minutes to 90 minutes after the morning dose, optionally wherein the morning dose is administered at least 30 minutes to 90 minutes before a meal.

10. The buffered liquid formulation for use according to any preceding claim, wherein:
i) a morning dose of the formulation and an evening dose of the formulation are administered, and wherein the evening dose is administered at least 6 hours after the morning dose; and/or
ii) the formulation is administered in the morning or in proximity to a first meal of the day, optionally wherein the formulation is administered at least 30 minutes to 90 minutes before the first meal of a day.

11. The buffered liquid formulation for use according to any preceding claim, wherein the number and/or severity of postprandial neuroglycopenic symptoms in the subject is reduced.

12. The buffered liquid formulation for use according to claim 11, wherein:
i) postprandial glucose nadir is improved for the subject, optionally wherein there is a significant increase the mixed meal tolerance test ("MMTT") glucose nadir for the subject, further optionally wherein the mean plasma glucose nadir during MMTT provocation increase by 21% - 25% for the subject compared to a control that is not administered the formulation; or
ii) the subject requires fewer glycemic rescues as compared to a control that is not administered the formulation.

13. The buffered liquid formulation for use according to any preceding claim, wherein one or more of the rate of hypoglycemia-induced CNS impairment, postprandial insulin peak, rate of severe hypoglycemia events, or postprandial glucose nadir is improved in the subject.

14. The buffered liquid formulation for use according to claim 13, wherein
i) postprandial insulin peak is reduced for the subject, optionally wherein mean postprandial insulin peak during an MMTT provocation is reduced from about 21% to 23% in the subject compared to a control that is not administered the formulation; or
ii) the rate of severe hypoglycemia events is reduced in the subject, optionally wherein a severe hypoglycemia event comprises symptoms requiring the subject to receive third party assistance; or
iii) the rate of hypoglycemia-induced CNS impairment is reduced in the subject.

15. The buffered liquid formulation for use according to any preceding claim, wherein:
a) peak GLP-1 and glucagon levels are higher in the subject as compared to a control that is not administered the formulation; and/or
b) GLP-1 and glucagon AUC values are higher during the MMTT in the subject as compared to a control that is not administered the formulation; and/or
c) the formulation is administered in a total volume of 0.25 ml to 2.0 ml; in a total volume of 0.05 ml 0.1 ml; or in an injection volume of 0.25 ml to 1.5 ml, 0.5 ml to 1 ml, 0.7 ml to 1 ml, or 0.05 ml to 0.1 ml; and/or
d) the subject is refractory to dietary treatment; and/or
e) the rate of rescue needed by the subject is reduced, optionally wherein rescue comprises administering glucagon to the subject at the earlier of glucose ≤ 50 mg/dL + neuroglycopenia or glucose ≤ 40 mg/dL +/- neuroglycopenia; and/or
f) the percent time in hypoglycemia by CGM threshold for the subject is reduced after administration; and/or
g) the number of episodes of hypoglycemia by CGM threshold for the subject is reduced after administration; and/or
h) there were fewer interruptions in daily activities for the subject as compared to a control that is not administered the formulation; and/or
i) the mean rates of hypoglycemia events in the subject are reduced from 30% to 60% for avexitide as compared to a control that is not administered the formulation, optionally wherein a hypoglycemia event comprises an SBGM glucose level <70 mg/dL; and/or
j) the mean rates of clinically important hypoglycemia in the subject are reduced by from 40% to 59% as compared to a control that is not administered the formulation, optionally wherein a clinically important hypoglycemia event comprises any neuroglycopenic symptoms/signs confirmed by SBGM glucose level <55 mg/dL or as a hypoglycemic episode that required assistance of another person to administer rescue therapy.
